# EUROPÄISCHE PATENTSCHRIFT

(11) **EP 2 489 344 B1**
(45) Veröffentlichungstag und Bekanntmachung des Hinweises auf die Patenterteilung: **24.03.2021**
(21) Anmeldenummer: 11154589.3
(22) Anmeldetag: 15.02.2011
(51) Int. Cl.: A61K 6/00

(54) **Dentalwerkstoff auf der Basis einer antimikrobiell wirksamen Verbindung**
Dental material based on an anti-microbial compound
Matériau dentaire comprenant une substance anti-microbienne

(43) Veröffentlichungstag der Anmeldung: 22.08.2012
(73) Patentinhaber: Ivoclar Vivadent AG, 9494 Schaan (LI)
(72) Erfinder: Salz, Ulrich, Dr., 88131 Lindau (DE); Bock, Thorsten, Dr., 6800 Feldkirch (AT); Fik, Christoph P., 77933 Lahr/schw. (DE); Tiller Jörg, Prof. Dr., 58313 Herdecke (DE); Rheinberger, Volker, Dr., 9490 Vaduz (LI)
(74) Vertreter: Uexküll & Stolberg

(56) Entgegenhaltungen:
- EP-A1- 1 849 450
- WO-A1-2009/103718
- DE-A1- 10 325 049
- DE-A1-102008 036 520
- US-A1- 2007 025 928
- US-A1- 2010 098 738
- WASCHINSKI ET AL.: "Influence of Satellite Groups on Telechelic Antimicrobial Functions of Polyoxazolines", MACROMOL. BIOSCI., Nr. 5, 2005, Seiten 149-156, XP002405169,

## Beschreibung

Die vorliegende Erfindung betrifft Dentalwerkstoffe auf der Basis einer Verbindung mit antimikrobieller Wirkung.

Die antimikrobielle Ausrüstung von Dentalwerkstoffen ist seit längerem bekannt und erfolgt meist durch rein physikalische Einmischung eines antimikrobiellen Wirkstoffs in eine entsprechende Werkstoffmatrix. So wird zum Beispiel in WO 98/48766 ein Zahnbeschichtungsmaterial zur Kariesprävention beschrieben, das als antimikrobiellen Wirkstoff Triclosan (2,4,4'-Trichlor-2'-hydroxydiphenylether) enthält. Auch in US 2003/0220416 werden Materialien für die zahnmedizinische Anwendung wie Prothesenkunststoffe und Befestigungszemente beschrieben, die antimikrobielle phenolische Substanzen wie Triclosan enthalten.

Die DE 198 13 686 offenbart ein Zahnwurzelfüllungsmaterial auf Guttaperchabasis, das als antimikrobiellen Wirkstoff Chlorhexidin freisetzt.

Die rein physikalische Einmischung antimikrobieller Wirkstoffe in einen Werkstoff ist mit mehreren Problemen behaftet. Um seine antimikrobielle Wirkung zu erreichen, muss der Wirkstoff über die Funktionsdauer des Werkstoffs freigesetzt werden. In der Regel sind die Freisetzungsraten der Art, dass der Wirkstoff initial in sehr hoher Konzentration freigesetzt wird und danach der antimikrobielle Effekt weitgehend verloren geht. Nachteilig ist dabei zudem, dass bei hohen Wirkstoffkonzentrationen toxische Nebenwirkungen auftreten können, was besonders bei medizinischen Anwendungen unerwünscht ist. Aus diesem Grund ist man besonders für derartige Anwendungen dazu übergegangen, den Wirkstoff polymerisierbar zu machen, um diesen bei der Aushärtung des Werkstoffs durch Homopolymerisation oder Copolymerisation mit einem anderen polymerisierbaren Monomeren im sich bildenden Polymeren zu immobilisieren.

US 5,536,861 und US 5,358,688 offenbaren Organosilikonmonomere für die Anwendung in Kontaktlinsen, die als antimikrobielle Gruppe eine quartäre Ammoniumgruppe enthalten.

EP 0 663 409 offenbart Monomere für die Anwendung in Kontaktlinsen, die als wirksame Gruppe quartäre Phosphoniumgruppen enthalten.

In EP 0 537 774 werden polymerisierbare Wirkstoffmonomere beschrieben, die eine quartäre Ammoniumgruppe enthalten. Als polymerisierbare Gruppe werden (Meth)acrylfunktionalitäten verwendet, zwischen der polymerisierbaren Gruppe und der wirksamen Gruppe befindet sich ein Alkylenspacer mit 2 bis 18 C-Atomen.

In EP 0 705 590 und WO 01/90251 werden spezielle Zusammensetzungen für die dentale Anwendung beschrieben, die ein antimikrobielles, polymerisierbares Monomer gemäß EP 0 537 774 enthalten.

Nachteilig bei der Anwendung von polymerisierbaren Wirkstoffmonomeren ist, dass meist nur das Monomer eine antimikrobielle Wirkung aufweist, die nach der Polymerisation verloren geht. Häufig ist nur vorhandenes Restmonomer für die antimikrobielle Wirkung der Polymeren verantwortlich, so dass nach der Eluierung der nicht polymerisierten antimikrobiellen Monomere die antimikrobielle Wirkung der Werkstoffe verblasst. Dadurch wird die antimikrobielle Langzeitwirkung deutlich reduziert, wodurch z.B. Arzneimittel oder Medizinprodukte ihre klinische Tauglichkeit zumindest teilweise verlieren.

Die EP 1 849 450 offenbart Dentalmaterialien, die mit einem antimikrobiell wirksamen Makromer ausgerüstet sind, bei dem eine antimikrobiell wirksame Gruppe über einen polymeren Spacer mit einer radikalisch polymerisierbaren Gruppe verbunden ist und das auch nach der Polymerisation eine hohe antimikrobielle Wirksamkeit zeigt.

US 6,316,015 beschreibt Substrate, an deren Oberflächen Moleküle mit antibiotischen, bakteriziden, viruziden oder fungiziden Eigenschaften kovalent gebunden sind, wie z.B. Katheter, Spritzen, Nadeln und Schläuche für medizinische Anwendungen.

Die EP 1 707 601 beschreibt eine Methode zur Behandlung von Oberflächen, bei der ein statistisches Copolymer an die Oberfläche gebunden wird, um dieser cytotoxische oder Zelladhäsions-Eigenschaften zu verleihen. Das statistische Copolymer besitzt mindestens eine Monomereinheit A, deren reaktive Stelle kovalente Bindungen mit dem Substrat eingehen kann, und eine Monomereinheit B, die mindestens ein antimikrobielles, antivirales oder fungizides Molekül umfasst. Die Methode soll zur antiseptischen Behandlung verschiedener Substrate, wie beispielsweise Kunststoffe, Holz, Papier oder Textilien anwendbar sein und insbesondere im Bereich Körperpflege, im klinischen Bereich sowie in Haushalt und Lebensmittelindustrie eingesetzt werden.

Der Erfindung liegt die Aufgabe zugrunde, Dentalmaterialien und Dentalwerkstoffe bereitzustellen, die eine dauerhafte, hohe antimikrobielle Wirkung haben und die genannten Nachteile nicht aufweisen.

Diese Aufgabe wird erfindungsgemäß durch Dentalwerkstoffe gelöst, die mindestens eine antimikrobiell wirksame Verbindung der Formel (I):

[AG]ₘ-R¹-Z-SP-Y-R²-[WG]ₚ (I),

in der die Variablen die folgenden Bedeutungen haben:
- m: = 1 oder 2;
- p: = 1 oder 2;
- R¹: = ein linearer oder verzweigter C₁ bis C₂₀-Alkylenrest, der ein- oder mehrfach durch O, S, NH, N⁺R³₂, SiR³₂, CONH, CONR³, COO und/oder OCONH unterbrochen sein kann;
- R²: = ein substituierter oder unsubstituierter, aromatischer C₆ bis C₁₄-Rest;
- R³: = jeweils unabhängig ein linearer oder verzweigter C₁ bis C₂₀-Alkylrest;
- AG: = eine Ankergruppe ausgewählt aus: 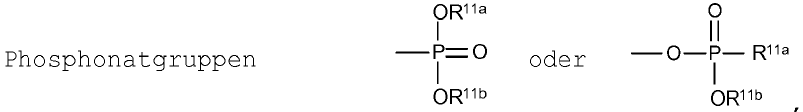 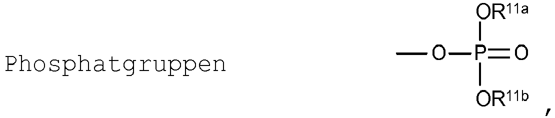
wobei R^{11a} und R^{11b} unabhängig ausgewählt sind aus H, Alkyl, Aryl oder -Si(Alkyl)₃, wobei Alkyl bevorzugt für Methyl, Ethyl, n-Propyl oder i-Propyl steht, und wobei besonders bevorzugt R^{11a} und R^{11b} jeweils Alkyl sind oder R^{11a} H und R^{11b} Alkyl ist, 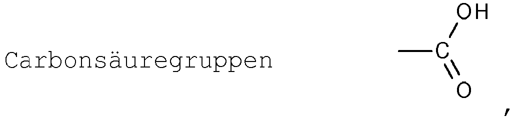 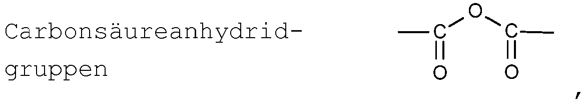
SP = ein polymerer Spacer, der aus Polyoxazolin-, Polyester-, Polyamid-, Polyharnstoff-, Polyurethan-, Polycyanoacrylat-, Polyacrylat- und Polymethacrylatgruppen ausgewählt ist;
WG = eine antimikrobiell wirksame Gruppe wie unten definiert;
Y = entfällt, 0, S oder COO;
Z = entfällt, 0, S, NH, N⁺R³₂ oder eine Amidgruppe,
mindestens ein radikalisch polymerisierbares Monomer und mindestens einen Initiator für die radikalische Polymerisation enthalten.

Allgemein enthalten die Verbindungen der Formel (I) gemäß der obigen Definition einen polymeren Spacer SP, der vorzugsweise jeweils endständig mit einer oder mehreren Ankergruppen AG und einer oder mehreren Wirkstoffgruppen WG verbunden ist.

Die bei R¹, R² und R³ gegebenenfalls vorhandenen Substituenten sind unabhängig voneinander vorzugsweise aus (C₁-C₆) Alkyl, (C₁-C₆)Alkoxy, Aryl, Benzyl, F, Cl, Br, I, OH, -COOH, -COO-(C₁-C₆)Alkyl, -CONH₂, -COR⁴ und -COOR⁴ ausgewählt, wobei R⁴ ein (C₁-C₂₀)Alkyl-, Phenyl- oder Benzylrest ist.

Durch die Formel (I) werden nur solche Verbindungen erfasst, die mit der chemischen Valenzlehre vereinbar sind. Der Hinweis, dass ein Rest z.B. durch O, S, NH, SiR³₂, CONH, CONR³, COO, OCONH etc. unterbrochen sein kann, ist so zu verstehen, dass diese Atome oder Gruppen in die Kohlenstoffkette des Restes eingeschoben werden, d.h. beidseitig von Kohlenstoffatomen begrenzt werden. Die Anzahl dieser Fremdatome oder Gruppen ist daher um mindestens 1 kleiner als die Zahl der Kohlenstoffatome, und die Fremdatome oder Gruppen können nicht endständig sein.

Unter Kombinationen von Alkylenresten und aromatischen Gruppen werden vorzugsweise Alkylen-Arylen-, Alkylen-Arylen-Alkylen- und Arylen-Alkylen-Arylen-Gruppen, insbesondere -CH₂-Ph- und -CH₂-Ph-CH₂- verstanden.

Sind in der Verbindung der Formel (I) mehrere Ankergruppen AG vorhanden (m = 2), so bilden die Ankergruppen zusammen mit Atomen des Rests R¹ mehrfunktionelle Ankergruppen. Dabei können die Ankergruppen AG gleich oder verschieden sein. In einer bevorzugten Ausführungsform sind die Ankergruppen gleich.

Beispiele für mehrfunktionelle Ankergruppen sind Dicarbonsäuregruppen, Bisphosphonat- und Bisphosphatgruppen, vorzugsweise Dialkyladipimidatgruppen.

Beispielshafte Carbonsäuregruppen sind:

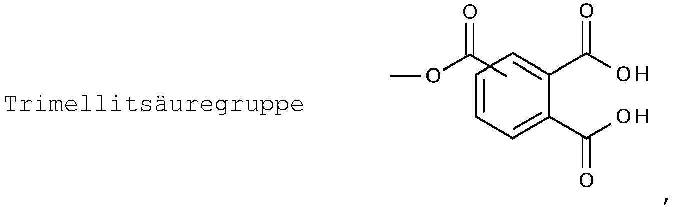

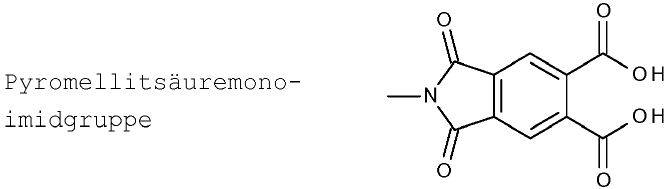

Beispielhafte Carbonsäureanhydridgruppen sind:

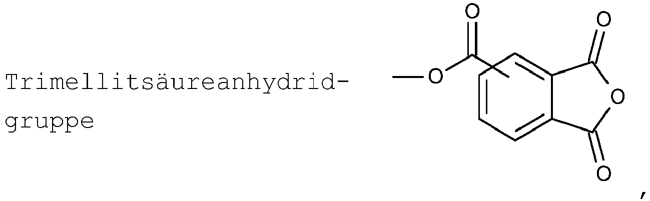

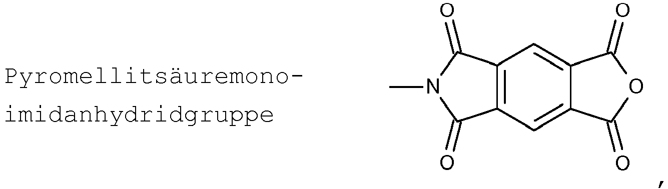

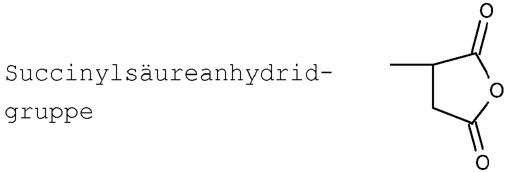

Sind in der Verbindung der Formel (I) mehrere antimikrobiell wirksame Gruppen WG vorhanden (p = 2), so können auch diese gleich oder verschieden sein. In einer bevorzugten Ausführungsform der Verbindung der Formel (I) sind die antimikrobiell wirksamen Gruppen WG gleich.

In einer weiteren bevorzugten Ausführungsform sind m = 1 und p = 1 und die Ankergruppe AG und Wirkstoffgruppe WG jeweils endständig.

Der Begriff polymerer Spacer umfasst hier allgemein Spacer mit mindestens 3, insbesondere 3 bis 500, vorzugsweise 5 bis 200, besonders bevorzugt 10 bis 100, am meisten bevorzugt 10 bis 50 Wiederholungseinheiten.

Die Verbindung der Formel (I) hat vorzugsweise ein Molekulargewicht von mindestens 250 g/mol, insbesondere mindestens 500 g/mol, vorzugsweise mindestens 1000 g/mol, besonders bevorzugt mindestens 1500 g/mol.

In einer weiteren bevorzugten Ausführungsform der erfindungsgemäßen Verbindung der Formel (I) ist R¹ und/oder R² ein linearer oder verzweigter C₅ bis C₂-Alkylenrest und besonders bevorzugt ein C₁₀ bis C₂₀-Alkylenrest.

AG ist ausgewählt aus Phosphonat-, Phosphat-, Carbonsäure- und Carbonsäureanhydridgruppen, bevorzugt Phosphonat-, insbesondere Dialkylphosphonat-, Phosphat-, Carbonsäure- und Carbonsäureanhydridgruppen, am meisten bevorzugt multifunktionelle Carbonsäuregruppen, insbesondere Dicarbonsäuregruppen, Carbonsäureanhydridgruppen und Phosphonatgruppen. Solche Ankergruppen sind besonders zur Modifizierung von Zahnschmelz, Dentin sowie Substraten auf Basis von Metalloxiden geeignet.

Die antimikrobielle Gruppe (WG) ist eine Pyridiniumgruppe der Formel 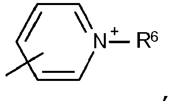 die in ortho-, meta- oder para-Position und besonders bevorzugt in para-Position mit dem Rest der Verbindung der Formel (I) verknüpft ist,
eine Pyridiniumgruppe der Formel 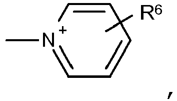 die in ortho-, meta- oder para-Position und besonders bevorzugt in para-Position mit R⁶ substituiert sein kann, wobei R⁶ jeweils unabhängig für H oder einen linearen oder verzweigten C₁ bis C₂₀-Alkylrest steht,
oder
WG = -N⁺R⁷R⁸R⁹ A⁻, mit
R⁷ = H, ein linearer oder verzweigter C₁ bis C₂₀-Alkylrest, vorzugsweise ein C₁ bis C₆-Alkylrest, besonders bevorzugt -CH₃;
R⁸ = H, ein linearer oder verzweigter C₁ bis C₂-Alkylrest, vorzugsweise ein C₁ bis C₆-Alkylrest, besonders bevorzugt -CH₃;
oder R⁷ und R⁸ bilden zusammen mit dem Stickstoffatom, an das sie gebunden sind, ein aromatisches oder nicht aromatisches Ringsystem oder einen aromatischen oder nicht aromatischen Ring, vorzugsweise 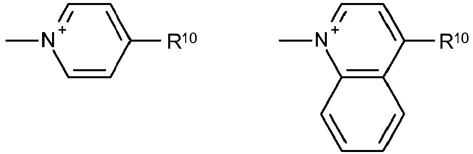 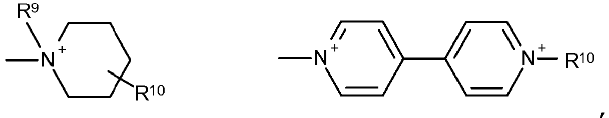 wobei R¹⁰ ein linearer oder verzweigter C₁ bis C₂₀-Alkylrest ist;
R⁹ = entfällt, H, ein linearer oder verzweigter C₁ bis C₃₁-Alkylrest, vorzugsweise -(CH₂)ᵣ-CH₃;
r = 5 bis 30, vorzugsweise 10 bis 25, besonders bevorzugt 10 bis 20,
oder
WG = -P⁺R^{7'}R^{8'}R^{9'} A⁻, mit
- R^{7'}: = H, ein linearer oder verzweigter C₁ bis C₂₀-Alkylrest, vorzugsweise ein C₁ bis C₆-Alkylrest, besonders bevorzugt -CH₃;
- R^{8'}: = H, ein linearer oder verzweigter C₁ bis C₂₀-Alkylrest, vorzugsweise -CH₃;
- R^{9'}: = entfällt, H, ein linearer oder verzweigter C₁ bis C₃₁-Alkylrest, vorzugsweise -(CH₂)ᵣ-CH₃;
- r: = 5 bis 30, vorzugsweise 10 bis 25, besonders bevorzugt 10 bis 20,
oder
WG = 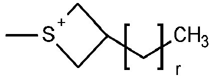 A⁻ mit
- r: = 5 bis 30, vorzugsweise 10 bis 25, besonders bevorzugt 10 bis 20,
oder WG ist eine quartäre Alkylphosphonium- oder Alkylsulfoniumgruppe, Octenidin oder ein Derivat davon, Chlorhexidin oder ein anderes Bisguanidin, Triclosan oder ein anderes Chlorphenol, eine anorganische Gruppe mit antimikrobiellen Eigenschaften, bevorzugt eine anorganische Gruppe, die ein oder mehrere antimikrobiell wirksame Metallionen wie z.B. Ag⁺, Cu²⁺, Sn²⁺, Sn⁴⁺, Zn²⁺ oder Al³⁺, beispielsweise eine Zinnoxofluoridgruppe (-O-SnF₃) enthält, eine antimikrobielle Peptidgruppe wie z.B. eine Magainin-Gruppe oder eine Lingual Antimicrobial Peptide (LPA)-Gruppe.

Die antimikrobiell wirksame Gruppe WG kann eine positive Ladung aufweisen. Diese wird durch Anionen A⁻ ausgeglichen. Bevorzugte Anionen A⁻ sind F⁻, Cl⁻, Br⁻, I⁻, Triflat (Trf), Mesylat (Mes), Tosylat (Tos), Palmitat, Stearat, Gluconat, Hexafluorophosphat, Phenolat, (Meth)acrylphosphonat, Phosphat, Hydrogenphosphat oder Dihydrogenphosphat, insbesondere F⁻, Cl⁻, Br⁻, I⁻, Triflat (Trf), Mesylat (Mes) oder Tosylat (Tos).

Insbesondere bevorzugt sind Verbindungen der Formel (I), in denen WG eine Pyridiniumgruppe der Formel 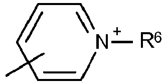 ist, die am quartären Stickstoff mit R⁶ = C₅ bis C₂₀-Alkyl substituiert ist. Am meisten bevorzugt ist dabei eine Cetylpyridiniumgruppe mit R⁶ = C₁₆-Alkyl, beispielsweise Cetylpyridiniumchlorid oder Cetylpyridiniumbromid.

Ebenso besonders bevorzugt sind Verbindungen der Formel (I), in denen WG eine quartäre Alkylammoniumgruppe der Formel 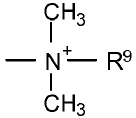 ist, wobei R⁹ C₁ bis C₃₁-Alkyl, vorzugsweise C₅ bis C₂₀-Alkyl, insbesondere C₇ bis C₁₈-Alkyl, besonders bevorzugt C₁₁ bis C₁₆-Alkyl und am meisten bevorzugt C₁₁ bis C₁₄-Alkyl ist. Am meisten bevorzugt ist WG eine N,N-Dimethyldodecylammoniumgruppe oder N,N-Dimethylcetylammoniumgruppe.

In einer bevorzugten Ausführungsform ist der Spacer SP eine Polyoxazolingruppe der Formel: 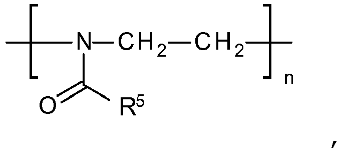 wobei n bevorzugt 3 bis 500, vorzugsweise 5 bis 200 und besonders bevorzugt 10 bis 100 ist und R⁵ bevorzugt -CH₃, -C₂H₅ oder -C₃H₇ ist.

Bevorzugte Definitionen der übrigen Variablen, die unabhängig voneinander gewählt werden können, sind:
- m: = 1;
- p: = 1;
- R¹: = ein C₁ bis C₁₀-Alkylenrest und am meisten bevorzugt -CH₂-CH₂- oder -CH₂-CH₂-NH-CH₂-CH₂-;
- R²: = Phenylen oder -CH₂-Ph-CH₂-;
- R³: = Methyl, Ethyl, n-Propyl oder i-Propyl, am meisten bevorzugt Methyl;

- Y: = entfällt;
- Z: = entfällt, NH, oder N⁺(CH₃)₂.

Besonders bevorzugt sind naturgemäß solche Verbindungen der Formel (I), bei denen alle Variablen eine der bevorzugten und insbesondere der besonders bevorzugten Bedeutungen haben.

Erfindungsgemäß besonders bevorzugte Verbindungen der Formel (I) sind: 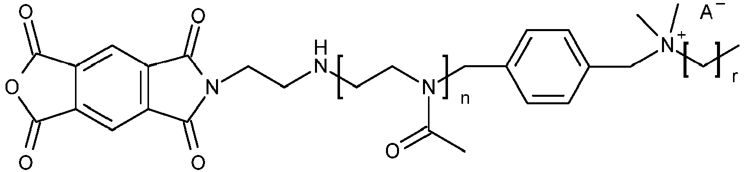 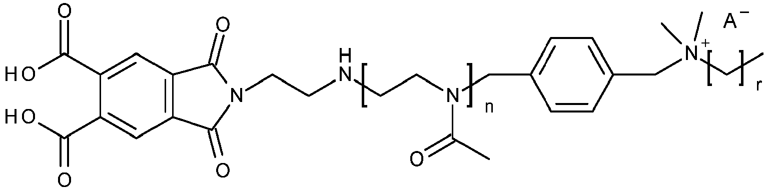 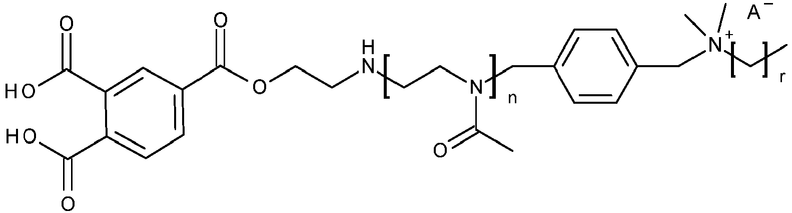 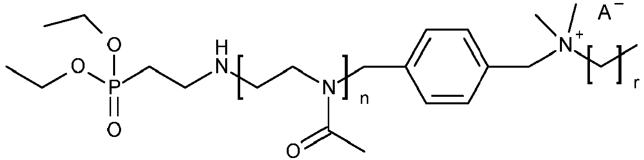 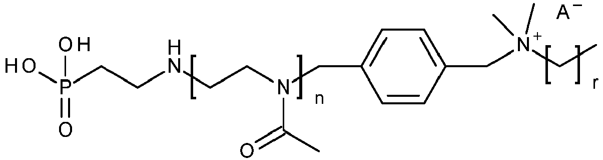 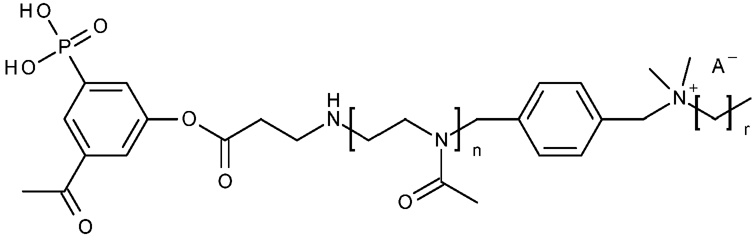 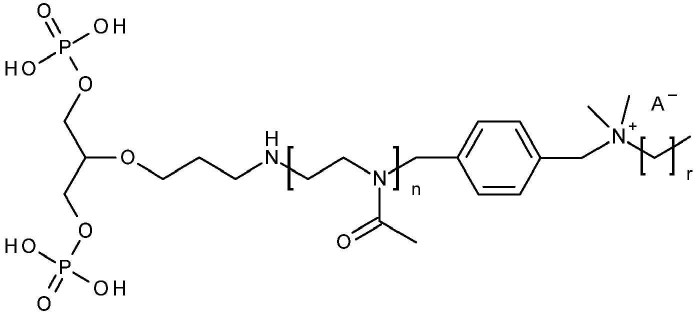 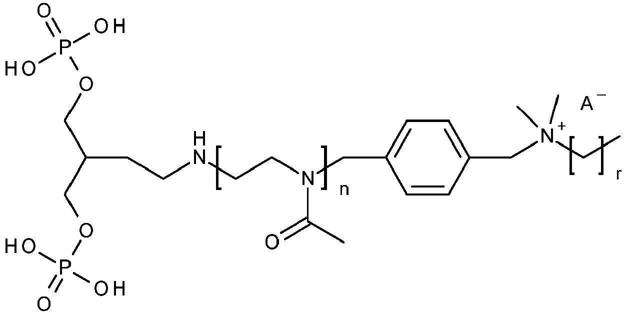 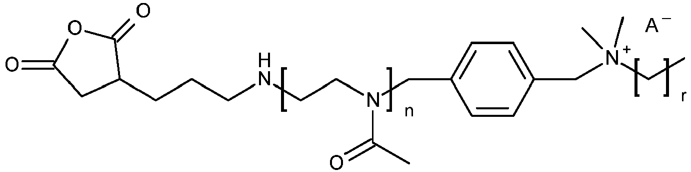 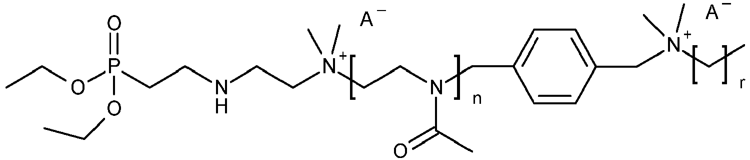 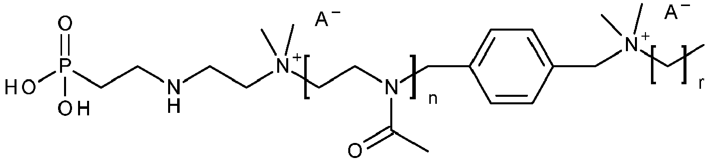 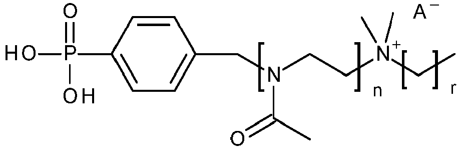 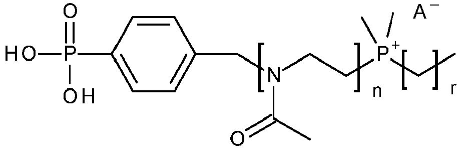 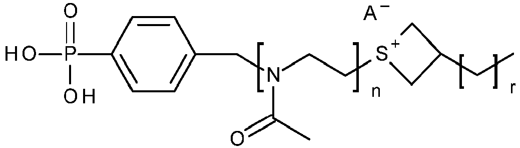 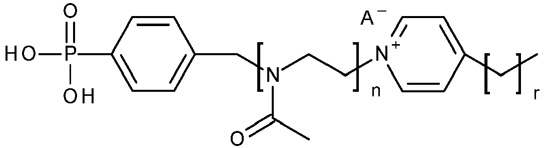 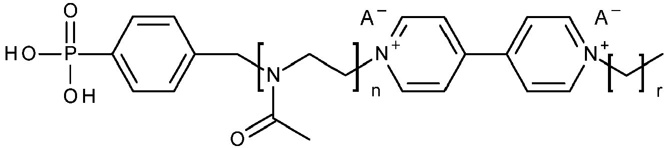 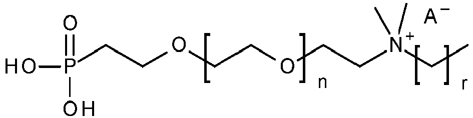 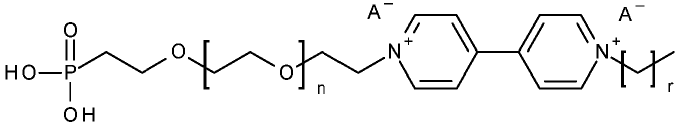 wobei
- A: = Cl, Br, I, Trf, Mes, Tos;
- n: = 3 bis 500;
- r: = 10 bis 20.

Die Verbindungen der Formel (I) lassen sich durch an sich bekannte mehrstufige Syntheseverfahren herstellen. Beispielsweise lassen sich die besonders bevorzugten Verbindungen der Formel (I), in denen SP eine Polyoxazolingruppe ist, auf die folgenden Weisen herstellen. Dabei wird der polymere Spacer SP durch Polymerisation von 2-Alkyl-1,3-oxazolin aufgebaut. Die Steuerung des Polymerisationsgrades und somit der Spacerlänge erfolgt über das Monomer-Initiator-Verhältnis [M₀]/[I] und die Reaktionszeit.

In einer ersten Synthesevariante wird als Initiator eine Verbindung verwendet, die eine antimikrobiell wirksame Gruppe WG enthält. Für den Polymerisationsabbruch wird vorzugsweise ein Amin verwendet, das eine Ankergruppe AG enthält. Alternativ kann für den Polymerisationsabbruch beispielsweise ein bifunktionelles Amin wie Ethylendiamin verwendet werden, dessen zweite funktionelle Gruppe anschließend zur Anknüpfung der Ankergruppe AG dient.

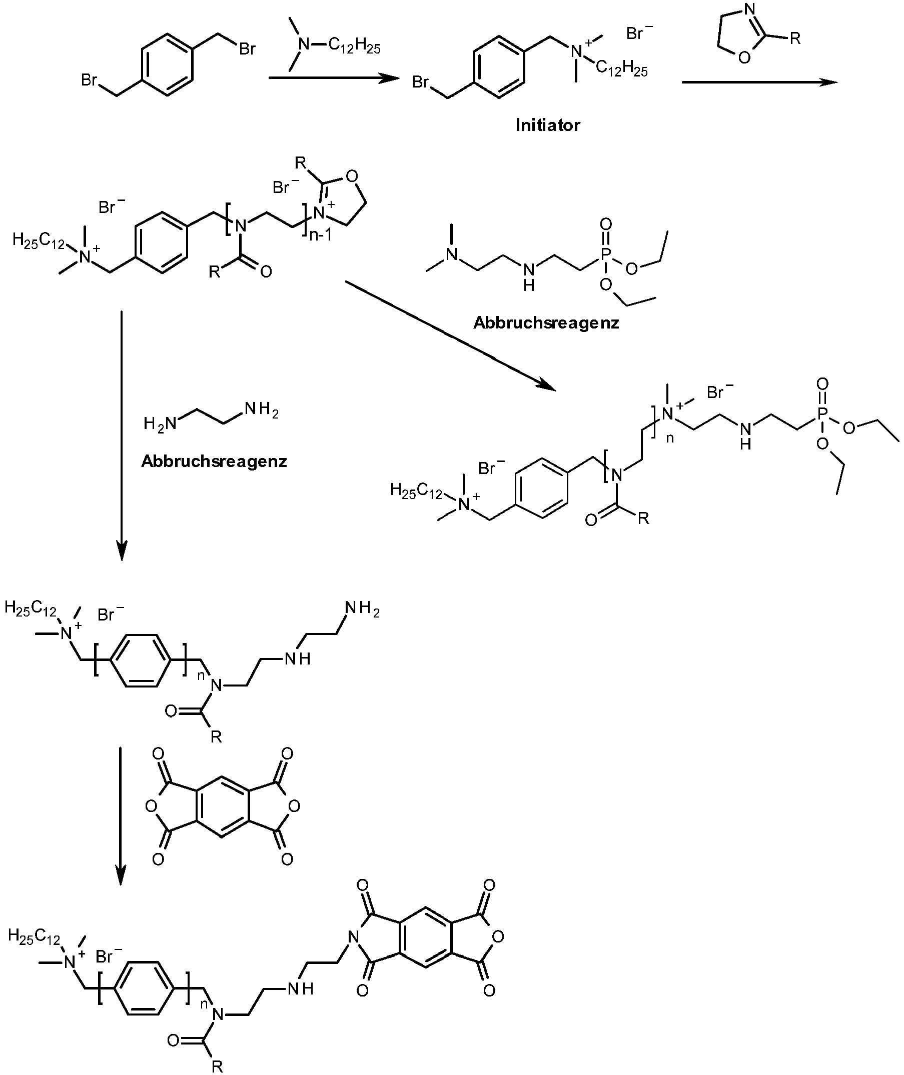

In einer zweiten Synthesevariante wird als Initiator eine Verbindung verwendet, die eine Ankergruppe AG enthält. Für den Polymerisationsabbruch wird vorzugsweise ein Amin verwendet, das eine antimikrobiell wirksame Gruppe WG enthält. Besonders bevorzugt wird für den Polymerisationsabbruch ein tertiäres Amin verwendet, wodurch sich als antimikrobiell wirksame Gruppe WG die entsprechende quartäre Ammoniumgruppe bildet.

Als Initiator kann auch eine Verbindung mit einer geeigneten Schutzgruppe verwendet werden. In diesem Fall wird je nach gewählter Synthesevariante die antimikrobiell wirksame Gruppe WG bzw. die Ankergruppe AG nach Abschluss der Polymerisation und Entfernung der Schutzgruppe eingeführt.

Die erfindungsgemäßen Dentalwerkstoffe enthalten
(a) mindestens eine antimikrobiell wirksame Verbindung der Formel (I) oder einen mit mindestens einer Verbindung der Formel (I) oberflächenmodifizierten Füllstoff;
(b) mindestens ein radikalisch polymerisierbares Monomer; und
(c) mindestens einen Initiator für die radikalische Polymerisation.

Bevorzugte Ausführungsformen der Verbindung der Formel (I) sind oben definiert.

Bevorzugte radikalisch polymerisierbare Monomere sind mono- oder mehrfach funktionelle (Meth)acrylate oder (Meth)acrylamide ((Meth)acrylverbindungen). Unter monofunktionellen (Meth)acrylverbindungen werden Verbindungen mit einer, unter mehrfach funktionellen (Meth)acrylverbindungen Verbindungen mit zwei oder mehr, vorzugsweise 2 bis 3 (Meth)acrylgruppen verstanden. Mehrfach funktionelle Monomere haben vernetzende Eigenschaften.

Bevorzugte monofunktionelle (Meth)acrylverbindungen sind kommerziell verfügbare monofunktionelle Monomere, wie Methyl-, Ethyl-, Butyl-, Benzyl-, Furfuryl- oder Phenyl(meth)acrylat sowie 2-Hydroxyethyl- oder -propyl(meth)acrylat.

Besonders bevorzugt sind hydrolysestabile Monomere wie hydrolysestabile Mono(meth)acrylate, z.B. Mesitylmethacrylat oder 2-(Alkoyxymethyl)acrylsäuren, z.B. 2-(Ethoxymethyl)acrylsäure, 2-(Hydroxymethyl)acrylsäure, N-mono- oder -disubstituierte Acrylamide, wie z.B. N-Ethylacrylamid, N,N-Dimethylacrylamid, N-(2-Hydroxyethyl)acrylamid oder N-Methyl-N-(2-hydroxyethyl)acrylamid, und N-monosubstituierte Methacrylamide, wie z.B. N-Ethylmethacrylamid oder N-(2-Hydroxyethyl)methacrylamid sowie außerdem N-Vinylpyrrolidon und Allylether. Diese Monomere sind bei Raumtemperatur flüssig und eignen sich daher auch als Verdünner.

Bevorzugte mehrfach funktionelle Monomere sind Bisphenol-A-di(meth)acrylat, Bis-GMA (ein Additionsprodukt aus Methacrylsäure und Bisphenol-A-diglycidylether), ethoxyliertes Bisphenol-A-di(meth)acrylat, UDMA (ein Additionsprodukt aus 2-Hydroxyethylmethacrylat und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)-acrylat, sowie Butandioldi(meth)acrylat, 1,10-Decandioldi(meth)acrylat oder 1,12-Dodecandioldi(meth)acrylat.

Besonders bevorzugt sind weiterhin hydrolysestabile Vernetzermonomere, wie z.B. vernetzende Pyrrolidone, wie z.B. 1,6-Bis-(3-vinyl-2-pyrrolidonyl)-hexan, oder kommerziell zugängliche Bisacrylamide wie Methylen- oder Ethylenbisacrylamid, Bis-(meth)acrylamide, wie z.B. N,N'-Diethyl-1,3-bis-(acrylamido)-propan, 1,3-Bis-(methacrylamido)-propan, 1,4-Bis-(acrylamido)-butan oder 1,4-Bis-(acryloyl)-piperazin, die durch Umsetzung aus den entsprechenden Diaminen mit (Meth)acrylsäurechlorid synthetisiert werden können.

Die erfindungsgemäßen Dentalwerkstoffe enthalten vorzugsweise auch mindestens ein radikalisch polymerisierbares, säuregruppenhaltiges Monomer. Säuregruppenhaltige Monomere werden im Folgenden auch als acide Monomere bezeichnet. Bevorzugte Säuregruppen sind Carbonsäuregruppen, Phosphonsäuregruppen, Phosphatgruppen und/oder Sulfonsäuregruppen, wobei diese Gruppen in der Säureform oder in Form eines Esters vorliegen können. Besonders bevorzugt sind Monomere mit Phosphonsäuregruppen oder Phosphatgruppen. Die Monomere können eine oder mehrere acide Gruppen aufweisen, bevorzugte sind Verbindungen mit 1 bis 2 aciden Gruppen.

Bevorzugte polymerisationsfähige Carbonsäuren sind Maleinsäure, Acrylsäure, Methacrylsäure, 2-(Hydroxymethyl)acrylsäure, 4-(Meth)acryloyloxyethyltrimellitsäure und das entsprechende Anhydrid, 10-Methacryloyloxydecylmalonsäure, N-(2-Hydroxy-3-methacryloyloxypropyl)-N-phenylglycin und 4-Vinylbenzoesäure.

Bevorzugte Phosphonsäuremonomere sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure, 2-[4-(Dihydroxyphosphoryl)-2-oxa-butyl]-acrylsäure und 2-[2-Dihydroxyphosphoryl)-ethoxymethyl]-acrylsäure-2,4,6-trimethyl-phenylester.

Bevorzugte acide polymerisationsfähige Phosphorsäureester sind 2-Methacryloyloxypropylmono- und -dihydrogenphosphat, 2-Methacryloyloxyethylmono- und -dihydrogenphosphat, 2-Methacryloyloxyethyl-phenyl-hydrogenphosphat, Dipentaerythritol-pentamethacryloyloxyphosphat, 10-Methacryloyloxydecyl-dihydrogenphosphat, Dipentaerythritolpentamethacryloyloxyphosphat, Phosphorsäuremono-(1-acryloyl-piperidin-4-yl)-ester, 6-(Methacrylamido)-hexyldihydrogenphosphat und 1,3-Bis-(N-acryloyl-N-propyl-amino)-propan-2-yl-dihydrogenphosphat.

Bevorzugte polymerisationsfähige Sulfonsäuren sind Vinylsulfonsäure, 4-Vinylphenylsulfonsäure oder 3-(Methacrylamido)-propylsulfonsäure.

Zur Initiierung der radikalischen Photopolymerisation werden vorzugsweise Acylphosphinoxide, Bisacylphosphinoxide, Benzophenon, Benzoin sowie deren Derivate oder α-Diketone oder deren Derivate wie 9,10-Phenanthrenchinon, 1-Phenyl-propan-1,2-dion, Diacetyl oder 4,4-Dichlorbenzil eingesetzt. Bevorzugt werden Campherchinon und 2,2-Methoxy-2-phenyl-acetophenon und besonders bevorzugt α-Diketone in Kombination mit Aminen als Reduktionsmittel, wie z.B. 4-(Dimethylamino)-benzoesäureester, N,N-Dimethylaminoethylmethacrylat, N,N-Dimethyl-sym.-xylidin oder Triethanolamin, verwendet. Auch finden Kombinationen aus unterschiedlichen Photoinitiatoren Anwendung.

Als Initiatoren für eine sogenannte chemische Härtung werden Redox-Initiatorkombinationen, wie z.B. Kombinationen von Benzoylperoxid mit N,N-Dimethylsym.-xylidin oder N,N-Dimethyl-p-toluidin, verwendet. Darüber hinaus sind auch Redoxsysteme bestehend aus Peroxid und Reduktionsmittel, wie z.B. Ascorbinsäure, Barbiturate oder Sulfinsäuren, besonders geeignet.

Weiterhin können die Dentalwerkstoffe zur Verbesserung der mechanischen Eigenschaften oder zur Einstellung der Viskosität Füllstoffe enthalten. Als Füllstoffe werden erfindungsgemäß partikuläre Füllstoffe bevorzugt. Ganz besonders bevorzugt sind organische oder anorganische Füllstoffpartikel, die eine mittlere Teilchengröße [bestimmt durch Transmissions- (10-80 nm), Rasterelektronenmikroskopie (50 nm bis 5 µm) bzw. Laserbeugung (0,1 bis 100 µm)] von 10 nm bis 50 µm, besonders bevorzugt 10 nm bis 30 µm und ganz besonders bevorzugt 10 nm bis 5 µm aufweisen.

Bevorzugte partikuläre Füllstoffe sind amorphe kugelförmige Materialien auf der Basis von Oxiden, wie ZrO₂ und TiO₂ bzw. Mischoxiden aus SiO₂, ZrO₂ und/oder TiO₂ mit einer mittleren Partikelgröße von 10 nm bis 1 µm, nanopartikuläre oder mikrofeine Füllstoffe, wie pyrogene Kieselsäure oder Fällungskieselsäure mit einer mittleren Partikelgröße von 10 nm bis 500 nm sowie Minifüllstoffe, wie Quarz-, Glaskeramik- oder Glaspulver mit einer mittleren Partikelgröße von 0,1 bis 5 µm, vorzugsweise 0,2 bis 3 µm und ganz besonders bevorzugt 0,4 bis 1,5 µm, sowie röntgenopake Füllstoffe, wie Ytterbiumtrifluorid oder nanopartikuläres Tantal(V)-oxid bzw. Bariumsulfat mit einer mittleren Partikelgröße von 10 nm bis 500 nm.

Der Dentalwerkstoff enthält mindestens eine Verbindung der Formel (I). Dabei sind Verbindungen der Formel (I) bevorzugt, bei denen die Ankergruppe AG ausgewählt ist aus Phosphonat-, Dialkylphosphonat-, Phosphat, Sulfonsäure-, Carbonsäureanhydrid- oder Carbonsäuregruppe ist. Am meisten bevorzugt ist eine Dicarbonsäure-, Carbonsäureanhydrid- oder Phosphonatgruppe.

Der Füllstoffanteil ist vorzugsweise mit polymerisationsfähigen Gruppen oberflächenmodifiziert, beispielsweise mit einem Alkoxysilan wie 3-(Methacryloyloxy)propyltrimethoxysilan (MPTMS).

Außerdem können die erfindungsgemäßen Dentalwerkstoffe ein oder mehrere weitere Additive enthalten, die vorzugsweise aus Stabilisatoren, Inhibitoren, Aromastoffen, Farbstoffen, Pigmenten, fluoridionenabgebenden Additiven, optischen Aufhellern, Weichmachern und/oder UV-Absorbern ausgewählt sind. Ein bevorzugter UV-Absorber ist 2-Hydroxy-4-methoxybenzophenon, bevorzugte Stabilisatoren sind 2,6-Di-tert-butyl-4-cresol und 4-Methoxyphenol.

Die erfindungsgemäßen Dentalwerkstoffe enthalten vorzugsweise:
(a) 0,05 bis 50 Gew.-%, bevorzugt 0,5 bis 25 Gew.-% und besonders bevorzugt 2 bis 10 Gew.-% antimikrobiell wirksame Verbindung der Formel (I);
(b) 5 bis 95 Gew.-%, bevorzugt 5 bis 85 Gew.-% und besonders bevorzugt 5 bis 70 Gew.-% radikalisch polymerisierbares Monomer; und
(c) 0,01 bis 5 Gew.-% und bevorzugt 0,1 bis 5 Gew.-% Initiator für die radikalische Polymerisation.

Neben den Komponenten (a) bis (c) enthalten die erfindungsgemäßen Dentalwerkstoffe vorzugsweise auch:
(d) 5 bis 60 Gew.-%, bevorzugt 5 bis 50 Gew.-% und besonders bevorzugt 5 bis 45 Gew.-% acides radikalisch polymerisierbares Monomer; und/oder
(e) 1 bis 90 Gew.-% und bevorzugt 1 bis 85 Gew.-% Füllstoff;
   und/oder
(f) 0,5 bis 99,95 Gew.-%, bevorzugt 0,5 bis 60 Gew.-% und besonders bevorzugt 1 bis 40 Gew.-% Lösungsmittel; und/oder
(g) 0,01 bis 5 Gew.-% und bevorzugt 0,01 bis 3,0 Gew.-% weitere Additive.

Die genaue Zusammensetzung der erfindungsgemäßen Dentalwerkstoffe richtet sich nach dem gewünschten Anwendungszweck. Die erfindungsgemäßen Dentalwerkstoffe eignen sich insbesondere als Füllungsmaterialien und ganz besonders als Beschichtungsmaterialien, Adhäsive, Primer, selbsthaftende und/oder selbstkonditionierende Befestigungszemente.

Füllungsmaterialien weisen vorzugsweise die folgende Zusammensetzung auf:
(a) 0,05 bis 35 Gew.-%, bevorzugt 0,5 bis 25 Gew.-% und besonders bevorzugt 2 bis 10 Gew.-% Verbindung der Formel (I);
(b) 15 bis 25 Gew.-% radikalisch polymerisierbares Monomer, vorzugsweise Vernetzermonomer, insbesondere Bis-GMA, Urethandimethacrylat und/oder ethoxyliertes Bis-GMA;
(c) 0,5 bis 2,5 Gew.-% Initiator, vorzugsweise ein Campherchinon/Amin-Photoinitiatorsystem, insbesondere 0,2 bis 0,6 Gew.-% Campherchinon und 0,3 bis 0,6 Gew.-% Amin-Coinitiator;
(d) 50 bis 80 Gew.-% Füllstoff, vorzugsweise 45 bis 65 Gew.-% feingemahlenen Glasfüller, insbesondere Barium- oder Strontiumglasfüller, und 5 bis 25 Gew.-% röntgenopaken Füller, insbesondere Ytterbiumtrifluorid;
(e) 0 bis 39 Gew.-% eines gemahlenen Präpolymers;
(f) 0 bis 0,5 Gew.-%, vorzugsweise 0,01 - 0,5 Gew.-% Farbpigmente.

Beschichtungsmaterialien weisen vorzugsweise die folgende Zusammensetzung auf:
(a) 0,05 bis 10 Gew.-%, bevorzugt 0,1 bis 5 Gew.-% und besonders bevorzugt 0,5 bis 2,5 Gew.-% Verbindung der Formel (I) ;
(b) 50 bis 90 Gew.-%, bevorzugt 65 bis 85 Gew.-% radikalisch polymerisierbares Monomer, vorzugsweise 60,0 - 70,0 Gew.-% Vernetzermonomer, insbesondere Triacrylat, und 8,0 bis 15,0 Gew.-% Verdünnermonomer, insbesondere Methylmethacrylat;
(c) 0,5 bis 2,5 Gew.-% Initiator, vorzugsweise ein Campherchinon/Amin-Photoinitiatorsystem, insbesondere 0,2 bis 0,6 Gew.-% Campherchinon und 0,3 bis 0,6 Gew.-% Amin-Coinitiator und 0,5 bis 1,2 Gew.-% 2,4,6-Trimethylbenzoyldiphenylphosphinoxid;
(d) 5,0 bis 15 Gew.-% Füllstoff, insbesondere SiO₂-Nanopartikel;
(e) 0 bis 10,0 Gew.-% organisches Lösungsmittel.

Adhäsive und Primer weisen vorzugsweise die folgende Zusammensetzung auf:
(a) 0,05 bis 10 Gew.-%, bevorzugt 0,1 bis 5 Gew.-% und besonders bevorzugt 0,5 - 1,5 Gew.-% Verbindung der Formel (I);
(b) 10 bis 20 Gew.-% Vernetzermonomer, insbesondere Glycerindimethacrylat (GDMA) oder Triethylenglycoldimethacrylat (TEGDMA);
(c) 30 bis 50 Gew.-% hydrophiles Monomer, vorzugsweise 2-Hydroxyethylmethacrylat (HEMA);
(d) 20 bis 30 Gew.-% acides Monomer, vorzugsweise Methacryloyloxydecyldihydrogenphosphat (MDP);
(e) 0,5 bis 2,5 Gew.-% Initiator, vorzugsweise ein Campherchinon/Amin-Photoinitiatorsystem, insbesondere 0,2 bis 0,6 Gew.-% Campherchinon und 0,3 bis 0,6 Gew.-% Amin-Coinitiator, insbesondere Ethyl-p-dimethylaminobenzoat;
(f) 5 bis 20 Gew.-% eines organischen Lösungsmittels, vorzugsweise Ethanol.

Selbsthaftende und/oder selbstkonditionierende Befestigungszemente weisen vorzugsweise die folgende Zusammensetzung auf:
Basispaste:
(a) 0,05 bis 20 Gew.-%, bevorzugt 0,1 bis 10 Gew.-% und besonders bevorzugt 0,5 - 5,0 Gew.-% Verbindung der Formel (I);
(b) 25 bis 40 Gew.-% radikalisch polymerisierbares Monomer, vorzugsweise Vernetzermonomer, insbesondere Triethylenglycoldimethacrylat (TEGDMA) oder Urethandimethacrylat (UDMA);
(c) 1 bis 3,5 Gew.-% Amin, vorzugsweise N,N-Di-(2-hydroxy)-p-toluidin; und
(d) 15 bis 70 Gew.-% Füllstoff, vorzugsweise 2 bis 5,5 Gew.-% SiO₂-Partikel, 5 bis 25 Gew.-% röntgenopaker Füller, insbesondere Ytterbiumtrifluorid, 10 bis 45 Gew.-% Splitterpolymerisat und 0 bis 45 Gew.-% feingemahlener Glasfüller, insbesondere Barium- oder Strontiumglasfüller.

Katalysatorpaste:
(a) 3 bis 8 Gew.-% acides Monomer, vorzugsweise Methacryloyloxydecyldihydrogenphosphat (MDP);
(b) 25 bis 37 Gew.-% radikalisch polymerisierbares Monomer, vorzugsweise Vernetzermonomer, insbesondere Triethylenglycoldimethacrylat (TEGDMA) oder Urethandimethacrylat (UDMA);
(c) 0,5 bis 3 Gew.-% Peroxid, vorzugsweise Dibenzoylperoxid;
(d) 0,5 bis 2,5 Gew.-% Campherchinon/Amin-Photoinitiatorsystem; und
(e) 20 bis 70 Gew.-% Füllstoff, vorzugsweise 5 bis 25 Gew.-% röntgenopaker Füllers, insbesondere Ytterbiumtrifluorid, 2 bis 5,5 Gew.-% SiO₂-Partikel, 10 bis 45 Gew.-% Splitterpolymerisat und 5 bis 45 Gew.-% feingemahlener Glasfüller, insbesondere Bariumglas-, Strontiumglasfüller.

Im Folgenden wird die Erfindung anhand von Beispielen näher erläutert.

### Beispiele

### Beispiel 1: Herstellung einer Verbindung der Formel (I) mit Polyalkyloxazolinspacer und Pyromellitsäuremonoimidanhydrid-Ankergruppe

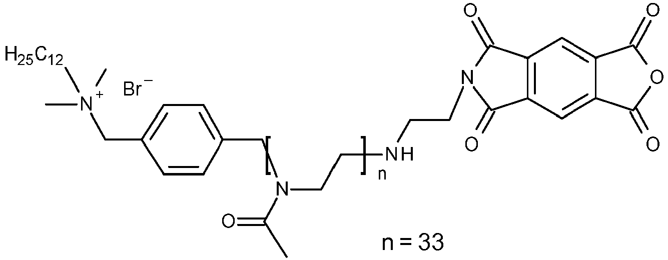

### 1.1. Herstellung der Starterverbindung 1 mit quartärer Alkylammoniumgruppe

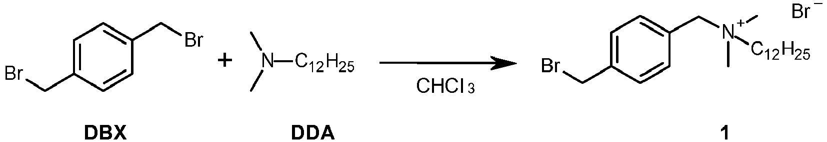

Die kommerziellen Edukte α,α'-Dibromo-*p*-xylol (DBX) und N,N-Dimethyldodecylamin (DDA) wurden vor ihrer Verwendung durch zweifache Umkristallisation aus CHCl₃ aufgereinigt. In einem Reaktionskolben wurde DBX (5,00 g, 18,94 mmol, 1,0 äq.) in kleinen Portionen unter starkem Rühren in trockenem CHCl₃ (100 ml) bei 25 °C gelöst. Anschließend wurde die Lösung so gerührt, dass gerade noch laminare Strömung im Kolben herrschte, und mittels Wasserbad auf 25 °C temperiert. In einem Tropftrichter (25 ml) wurde DDA (2,565 ml, 9,47 mmol, 0,5 äq.) mit trockenem CHCl₃ auf insgesamt 10,0 ml verdünnt. Das verdünnte DDA wurde sehr langsam binnen 45 min zu der Lösung von DBX zugetropft und der Ansatz weitere 15 min gerührt. Die Reaktionsmischung wurde am Rotationsverdampfer auf ca. 30 % v/v eingeengt und der gegebenenfalls ausfallende Feststoff (Edukt, Kleinstmenge) mittels Filtration abgetrennt. Der verbliebene klare Überstand wurde unter Rühren in THF (200 ml) gegeben und die resultierende Dispersion wurde mittels Zentrifugation (10 min, 5.000 U/min) separiert. Der sedimentierte weiße Feststoff (Nebenprodukt) wurde verworfen. Die homogene THF-Phase wurde auf ca. 50 % v/v eingeengt und die obige Prozedur insgesamt fünfmal durchgeführt. Nach Entfernung des restlichen Lösungsmittels wurde das Reinprodukt **1** als eine gelblich-braune, hochviskose Substanz erhalten.

### 1.2. Polymerisation von Methyloxazolin durch Initiierung mit Starterverbindung 1 und Terminierung mit Ethylendiamin 2

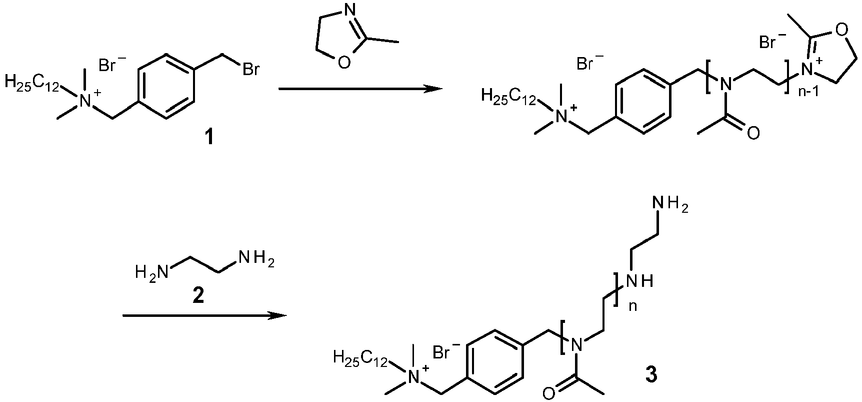

Die Starterverbindung (**1**, 0,56 g, 1,17 mmol, 1,0 äq.) wurde in trockenem CHCl₃ (18 ml, 20 °C, [Ar]) gelöst und in einen druckfesten Glasreaktor (50 ml) überführt. Methyloxazolin (3,00 g, 3,00 ml, 35,2 mmol, 30,0 äq.) wurde zugegeben. Die Polymerisation (4 h) erfolgte unter dielektrischem Heizen (100 °C) in einer Polymerisations-Mikrowelle (300 W). Die Reaktionsmischung wurde mit Ethylendiamin (**2**, 1,41 g, 1,57 ml, 23,4 mmol, 20,0 äq.) versetzt und im Wasserbad gerührt (24 h, 40 °C). Das aminfunktionalisierte Polymer wurde durch fünfmaliges Umfällen aus CHCl₃ in Diethylether als gelbliches, hygroskopisches Pulver erhalten (**3**, DP_{NMR}=33, 3,35 g, 93 %). Die Charakterisierung erfolgte mittels ¹H- und ¹³C-NMR sowie GPC.

### 1.3. Polymeranaloge Umsetzung von Poly(methyloxazolin) 3 mit der Pyromellit-Ankergruppe in DMF

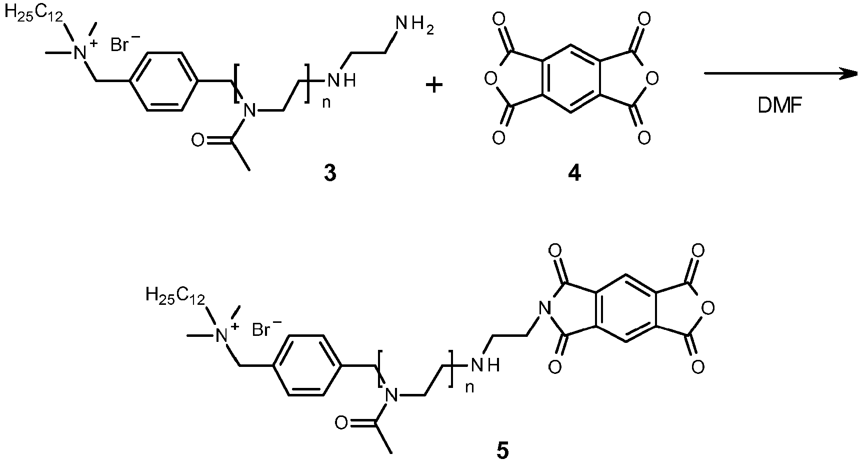

Pyromellitsäure-Dianhydrid (**4,** 3,96 g, 18,2 mmol, 20,0 äq.) wurde in DMF (60 ml, 30 °C) gelöst und mittels Eisbad auf 0 °C abgekühlt. Das aminterminierte Poly(methyloxazolin) **3** (3,00 g, 0,91 mmol, 1,0 äq.) wurde in trockenem DMF (40 ml, 0 °C, Restfeuchtigkeit < 3 ppm nach Destillation/Trocknung über basischem Aluminiumoxid) gelöst. Die Polymerlösung wurde der vorgelegten Verbindung **4** zügig zugetropft. Der Ansatz wurde 5 min lang gerührt. Das umgesetzte Polymer wurde durch fünfmaliges Umfällen aus DMF in Diethylether als gelb-bräunliches, hygroskopisches Pulver erhalten (**5,** DP_{NMR}=33, 2,95 g, 92 %). Die Charakterisierung erfolgte mittels ¹H-, ¹³C-NMR sowie GPC.

### Beispiel 2: Herstellung einer Verbindung der Formel (I) mit Polyalkyloxazolinspacer und Pyromellitsäuremonoimid-Ankergruppe

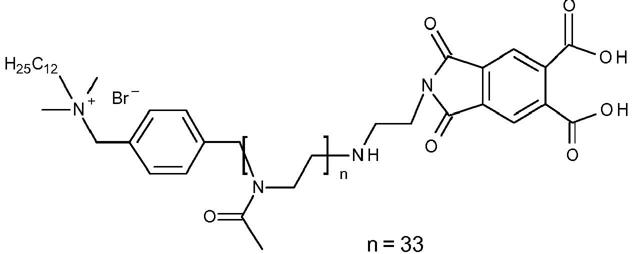

Durch Hydrolyse des Anhydrids **5** aus Beispiel 1 wurde die entsprechende Dicarbonsäure erhalten.

Die Hydrolyse erfolgte durch Rühren in destilliertem Wasser bei Raumtemperatur. Das Produkt wurde durch Einengen der Lösung und Sprühtrocknen oder Fällung in der Kälte gewonnen.

### Beispiel 3: Herstellung einer Verbindung der Formel (I) mit Polyalkyloxazolinspacer und Dialkylphosphonat-Ankergruppe

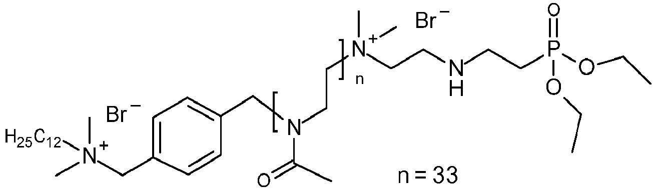

### 3.1. Herstellung des Terminierungagens 8

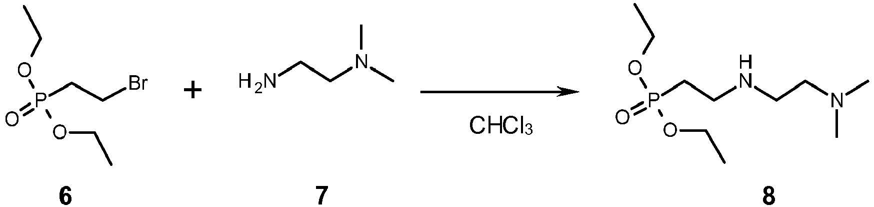

Diethyl-2-bromoethylphosphonat (**6**, 10,0 g, 40,8 mmol, 1,0 äq.) wurde in trockenem CHCl₃ (10 ml, 20 °C) gelöst. Anschließend wurde *N*,*N*-Dimethylethan-1,2-diamin (**7**, 3,60 g, 40,8 mmol, 1,0 äq.) in CHCl₃ (10 ml, 20 °C) 15 min langsam unter Rühren zugetropft.

Der Reaktionsansatz wurde in Diethylether ausgefällt und das so erhaltene Rohprodukt durch dreimaliges Umfällen aus Methanol in Diethylether aufgereinigt. Das Reinprodukt wurde als gelb-weiße, pastöse Substanz erhalten (**8**, 9,1 g, 88%). Die Charakterisierung erfolgte mittels ¹H- und ¹³C-NMR.

### 3.2. Polymerisation von Methyloxazolin, Initiierung durch Starterverbindung 1, Terminierung mit Aminophosphonat 8

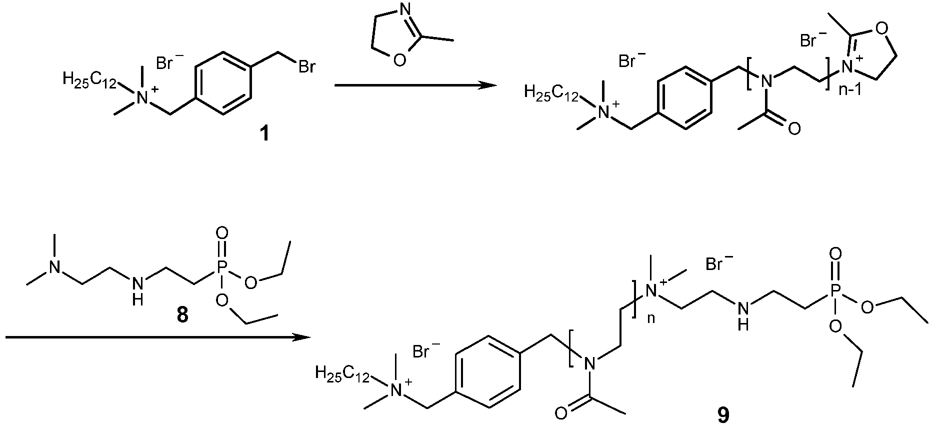

Die Starterverbindung aus Beispiel 1 (**1**, 0,56 g, 1,17 mmol, 1,0 äq.) wurde in trockenem CHCl₃ (18 ml, 20 °C, [Ar]) gelöst und in einen druckfesten Glasreaktor (50 ml) überführt. Methyloxazolin (3,00 g, 3,00 ml, 35,2 mmol, 30,0 äq.) wurde zugegeben. Die Polymerisation (4 h) erfolgte unter dielektrischem Heizen (100 °C) in einer Polymerisations-Mikrowelle (300 W).

Die Reaktionsmischung wurde mit dem Terminierungsagens (**8**, 5,90 g, 23,4 mmol, 20,0 äq.) versetzt, mit weiterem CHCl₃ (10 ml) verdünnt und im Wasserbad gerührt (48 h, 40 °C).

Das Aminophosphonat-funktionalisierte Polymer wurde durch fünfmaliges Umfällen aus CHCl₃ in Diethylether als gelbliches, hygroskopisches Pulver erhalten (**9**, DP_{NMR}=33, 3,95 g, 95 %). Die Charakterisierung erfolgte mittels ¹H-, ¹³C-NMR sowie GPC.

## Patentansprüche

1. Dentalwerkstoff, der
(a) mindestens eine antimikrobiell wirksame Verbindung der Formel (I)
[AG]ₘ⁻R ¹-Z-SP-Y-R²-[WG]ₚ (I),
in der die Variablen die folgenden Bedeutungen haben:
m = 1 oder 2;
p = 1 oder 2;
R¹ = ein linearer oder verzweigter C₁ bis C₂₀-Alkylenrest, der ein- oder mehrfach durch 0, S, NH, N⁺R³₂, SiR³₂, CONH, CONR³, COO und/oder OCONH unterbrochen sein kann;
R² = ein substituierter oder unsubstituierter, aromatischer C₆ bis C₁₄-Rest;
R³ = jeweils unabhängig ein linearer oder verzweigter C₁ bis C₂₀-Alkylrest;
AG = eine Ankergruppe ausgewählt aus: 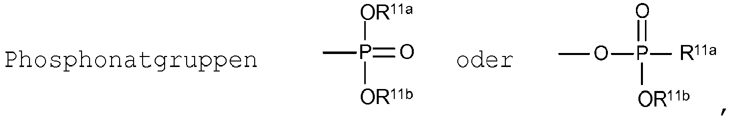 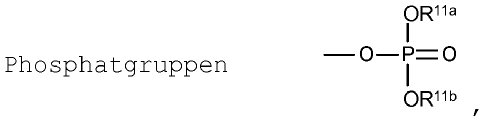
wobei R^{11a} und R^{11b} unabhängig ausgewählt sind aus H, Alkyl, Aryl oder -Si(Alkyl)₃, wobei Alkyl bevorzugt für Methyl, Ethyl, n-Propyl oder i-Propyl steht, und wobei besonders bevorzugt R^{11a} und R^{11b} jeweils Alkyl sind oder R^{11a} H und R^{11b} Alkyl ist, 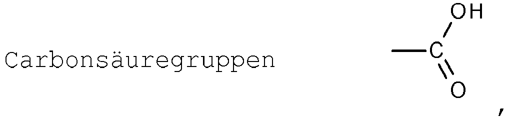 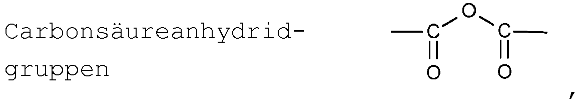 SP = ein polymerer Spacer, der aus Polyoxazolin-, Polyester-, Polyamid-, Polyharnstoff-, Polyurethan-, Polycyanoacrylat-, Polyacrylat- und Polymethacrylatgruppen ausgewählt ist;
WG = eine antimikrobiell wirksame Gruppe, ausgewählt aus
einer Pyridiniumgruppe der Formel 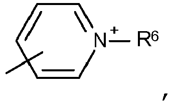 die in ortho-, meta- oder para-Position und besonders bevorzugt in para-Position mit dem Rest der Verbindung der Formel (I) verknüpft ist, oder
einer Pyridiniumgruppe der Formel 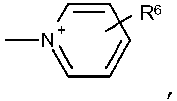 die in ortho-, meta- und para-Position und besonders bevorzugt in para-Position mit R⁶ substituiert sein kann,
wobei R⁶ jeweils unabhängig für H oder einen linearen oder verzweigten C₁ bis C₂₀-Alkylrest steht;
-N⁺R⁷R⁸R⁹ A⁻, mit
R⁷ = H, ein linearer oder verzweigter C₁ bis C₂₀-Alkylrest;
R⁸ = H, ein linearer oder verzweigter C₁ bis C₂₀-Alkylrest;
oder R⁷ und R⁸ bilden zusammen mit dem Stickstoffatom, an das sie gebunden sind, ein heteroaromatisches Ringsystem oder einen aromatischen oder nicht aromatischen Ring;
R⁹ = entfällt, H, ein linearer oder verzweigter C₁ bis C₃₁-Alkylrest;
A⁻ = ein Anion;
-P⁺R^{7'}R^{8'}R^{9'} A⁻, mit
R^{7'} = H, ein linearer oder verzweigter C₁ bis C₂₀-Alkylrest;
R^{8'} = H, ein linearer oder verzweigter C₁ bis C₂-Alkylrest;
R^{9'} = entfällt, H, ein linearer oder verzweigter C₁ bis C₃₁-Alkylrest;
A⁻ = ein Anion; 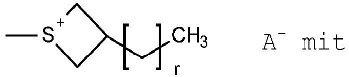
r = 5 bis 30;
A⁻ = ein Anion; oder
einer quartäre Alkylphosphonium- oder Alkylsulfoniumgruppe, Octenidin oder ein Derivat davon, Chlorhexidin oder ein anderes Bisguanidin, Triclosan oder ein anderes Chlorphenol, eine anorganische Gruppe mit antimikrobiellen Eigenschaften, eine antimikrobielle Peptidgruppe;
Y = entfällt, 0, S oder COO;
Z = entfällt, 0, S, NH, N⁺R³₂ oder eine Amidgruppe;
(b) mindestens ein radikalisch polymerisierbares Monomer;
und
(c) mindestens einen Initiator für die radikalische Polymerisation enthält.

2. Dentalwerkstoff nach Anspruch 1, bei dem R¹ ein linearer oder verzweigter C₅ bis C₂₀-Alkylenrest und bevorzugt ein C₁₀ bis C₂₀-Alkylenrest ist.

3. Dentalwerkstoff nach Anspruch 1 oder 2, bei dem AG eine Carbonsäuregruppe der Formel 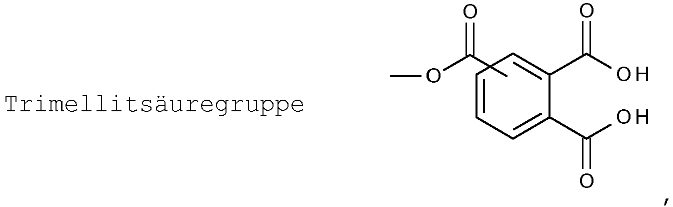 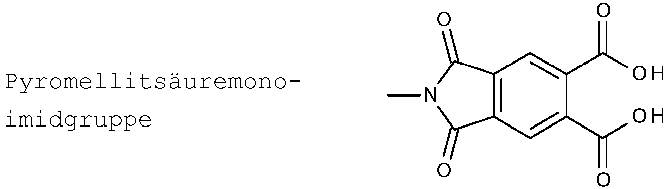 ist.

4. Dentalwerkstoff nach einem der Ansprüche 1 bis 3, bei dem AG eine Carbonsäureanhydridgruppe der Formel 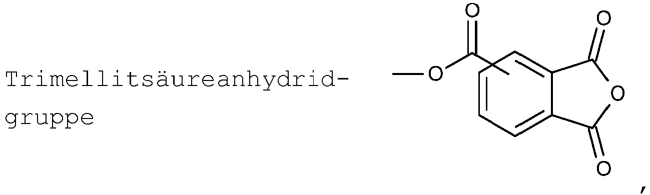 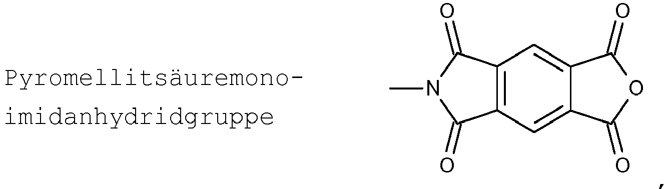 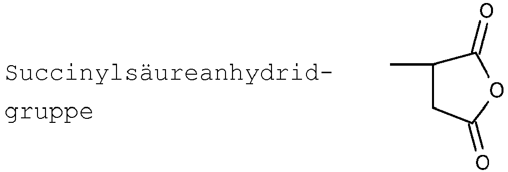 ist.

5. Dentalwerkstoff nach einem der Ansprüche 1 bis 4, bei dem die antimikrobiell wirksame Gruppe WG eine quartäre Alkylammoniumgruppe der Formel 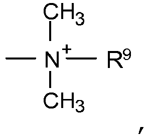 wobei R⁹ C₁ bis C₃₁-Alkyl, vorzugsweise C₅ bis C₂₀-Alkyl, insbesondere C₇ bis C₁₈-Alkyl, besonders bevorzugt C₁₁ bis C₁₆-Alkyl und am meisten bevorzugt C₁₁ bis C₁₄-Alkyl ist, und am meisten bevorzugt eine N,N-Dimethyldodecylammoniumgruppe oder N,N-Dimethylcetylammoniumgruppe ist.

6. Dentalwerkstoff nach einem der Ansprüche 1 bis 5, bei dem der Spacer SP eine Polyoxazolingruppe der Formel: 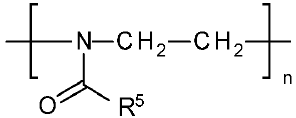 ist, wobei n bevorzugt 3 bis 500, vorzugsweise 5 bis 200 und besonders bevorzugt 10 bis 100 ist und R⁵ bevorzugt -CH₃, -C₂H₅ oder -C₃H₇ ist.

7. Dentalwerkstoff nach einem der Ansprüche 1 bis 6, bei dem mindestens eine der Variablen eine der folgenden Bedeutungen hat:
m = 1;
p = 1;
R¹ = ein C₁ bis C₁₀-Alkylenrest und am meisten bevorzugt -CH₂-CH₂- oder -CH₂-CH₂-NH-CH₂-CH₂-;
R² = Phenylen oder -CH₂-Ph-CH₂-;
R³ = Methyl, Ethyl, n-Propyl oder i-Propyl, am meisten bevorzugt Methyl;
SP = eine Polyoxazolin-, Polyester-, Polyamid-, Polyharnstoff-, Polyurethan-, Polycyanoacrylat-, Polyacrylat- oder Polymethacrylatgruppe;
AG = eine Phosphonat-, Phosphat-, Carbonsäure- oder Carbonsäureanhydridgruppe;
WG = eine Pyridiniumgruppe der Formel 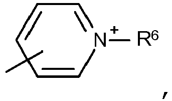
die in ortho-, meta- oder para-Position und besonders bevorzugt in para-Position mit dem Rest der Verbindung der Formel (I) verknüpft ist, oder
eine Pyridiniumgruppe der Formel 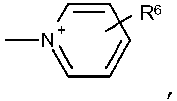 die in ortho-, meta- und para-Position und besonders bevorzugt in para-Position mit R⁶ substituiert sein kann, wobei R⁶ jeweils unabhängig für H oder einen linearen oder verzweigten C₁ bis C₂₀-Alkylrest steht;
-N⁺R⁷R⁸R⁹ A⁻,
mit
R⁷ = ein C₁ bis C₆-Alkylrest, bevorzugt -CH₃;
R⁸ = ein C₁ bis C₆-Alkylrest, bevorzugt -CH₃;
oder R⁷ und R⁸ bilden zusammen mit dem Stickstoffatom, an das sie gebunden sind, ein heteroaromatisches Ringsystem oder einen aromatischen oder nicht aromatischen Ring der Formel 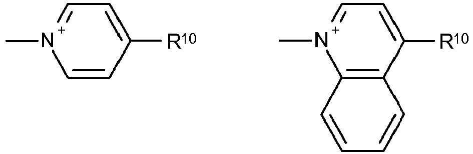 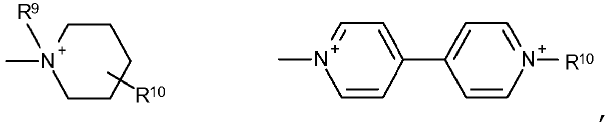 wobei R¹⁰ ein linearer oder verzweigter C₁ bis C₂₀-Alkylrest ist;
R⁹ = -(CH₂)ᵣ-CH₃;
r = 5 bis 30, vorzugsweise 10 bis 25, besonders bevorzugt 10 bis 20;
A⁻ = F⁻, Cl⁻, Br⁻, I⁻, Triflat (Trf), Mesylat (Mes), Tosylat (Tos), Palmitat, Stearat, Gluconat, Hexafluorophosphat, Phenolat, (Meth)acrylphosphonat, Phosphat, Hydrogenphosphat oder Dihydrogenphosphat, vorzugsweise F⁻, Cl⁻, Br⁻, I⁻, Triflat (Trf), Mesylat (Mes) oder Tosylat (Tos);
-P⁺R^{7'}R^{8'}R^{9'} A⁻,
mit
R^{7'} = ein C₁ bis C₆-Alkylrest, bevorzugt -CH₃;
R^{8'} = -CH₃;
R^{9'} = -(CH₂)ᵣ-CH₃;
r = 5 bis 30, vorzugsweise 10 bis 25, besonders bevorzugt 10 bis 20;
A⁻ = F⁻, Cl⁻, Br⁻, I⁻, Triflat (Trf), Mesylat (Mes), Tosylat (Tos), Palmitat, Stearat, Gluconat, Hexafluorophosphat, Phenolat, (Meth)acrylphosphonat, Phosphat, Hydrogenphosphat oder Dihydrogenphosphat, vorzugsweise F⁻, Cl⁻, Br⁻, I⁻, Triflat (Trf), Mesylat (Mes) oder Tosylat (Tos); 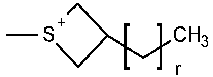
A mit
r = 10 bis 30, bevorzugt 10 bis 20;
A⁻ = F⁻, Cl⁻, Br⁻, I⁻, Triflat (Trf), Mesylat (Mes), Tosylat (Tos), Palmitat, Stearat, Gluconat, Hexafluorophosphat, Phenolat, (Meth)acrylphosphonat, Phosphat, Hydrogenphosphat oder Dihydrogenphosphat, vorzugsweise F⁻, Cl⁻, Br⁻, I⁻, Triflat (Trf), Mesylat (Mes) oder Tosylat (Tos); oder
eine quartäre Alkylphosphonium- oder Alkylsulfoniumgruppe, Octenidin oder ein Derivat davon, Chlorhexidin oder ein anderes Bisguanidin, Triclosan oder ein anderes Chlorphenol, eine anorganische Gruppe mit antimikrobiellen Eigenschaften, die ein oder mehrere antimikrobiell wirksame Metallionen enthält, eine antimikrobielle Magainin-Gruppe oder eine Lingual Antimicrobial Peptide (LPA)-Gruppe;
Y = entfällt;
Z = entfällt, NH, oder N⁺(CH₃)₂;
wobei alle Variablen unabhängig voneinander ausgewählt sein können.

8. Dentalwerkstoff nach einem der Ansprüche 1 bis 7, der
(a) 0,05 bis 50 Gew.-%, bevorzugt 0,5 bis 25 Gew.-% und besonders bevorzugt 2 bis 10 Gew.-% antimikrobiell wirksame Verbindung der Formel (I);
(b) 5 bis 95 Gew.-%, bevorzugt 5 bis 85 Gew.-% und besonders bevorzugt 5 bis 70 Gew.-% radikalisch polymerisierbares Monomer; und
(c) 0,01 bis 5 Gew.-% und bevorzugt 0,1 bis 5 Gew.-% Initiator für die radikalische Polymerisation enthält.

9. Dentalwerkstoff nach Anspruch 8, der zusätzlich
(d) 5 bis 60 Gew.-%, bevorzugt 5 bis 50 Gew.-% und besonders bevorzugt 5 bis 45 Gew.-% acides radikalisch polymerisierbares Monomer und/oder
(e) 1 bis 90 Gew.-% und bevorzugt 1 bis 85 Gew.-% Füllstoff und/oder
(f) 0,5 bis 99,95 Gew.-%, bevorzugt 0,5 bis 60 Gew.-% und besonders bevorzugt 1 bis 40 Gew.-% Lösungsmittel und/oder
(g) 0,01 bis 5 Gew.-% und bevorzugt 0,01 bis 3,0 Gew.-% weitere Additive enthält.

10. Verwendung eines Dentalwerkstoffs gemäß einem der Ansprüche 1 bis 9 zur Herstellung eines Adhäsivs, Primers, Zements, Beschichtungsmaterials oder Füllungsmaterials.

## Claims

1. Dental material, **characterised in that** it comprises
(a) at least one antimicrobially active compound of Formula (I)
[AG]ₘ-R¹-Z-SP-Y-R²-[WG]ₚ (I),
wherein the variables have the following meanings:
m = 1 or 2;
p = 1 or 2;
R¹ = a linear or branched C₁ to C₂₀ alkylene group that may be interrupted once or more by O, S, NH, N⁺R³₂, SiR³₂, CONH, CONR³, COO and/or OCONH;
R² = a substituted or unsubstituted, aromatic C₆ to C₁₄ group;
R³ = each independently a linear or branched C₁ to C₂₀ alkyl group;
AG = an anchor group selected from: 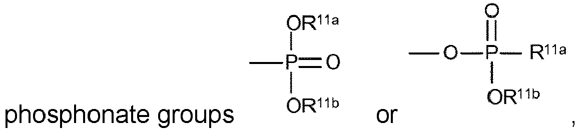 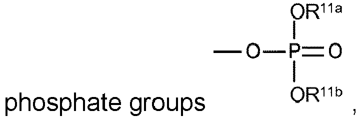 wherein R^{11a} and R^{11b} are independently selected from H, alkyl, aryl or-Si(alkyl)₃, wherein alkyl preferably stands for methyl, ethyl, *n*-propyl or *i-*propyl, and wherein particularly preferably R^{11a} and R^{11b} are each alkyl or R^{11a} is H and R^{11b} is alkyl, 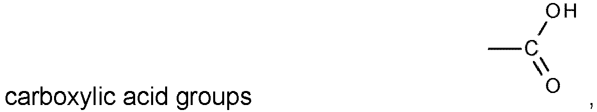 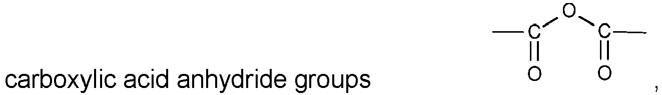
SP = a polymeric spacer that is selected from polyoxazoline, polyester, polyamide, polyurea, polyurethane, polycyanoacrylate, polyacrylate and polymethacrylate groups;
WG = an antimicrobially active group, selected from a pyridinium group of the Formula 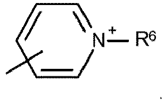 which is linked in the *ortho, meta* or para position and preferably in the *para* position with the remainder of the compound of Formula (I), or
a pyridinium group of the Formula 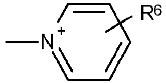 which may be substituted with R⁶ in the *ortho, meta* or *para* position and particularly preferably in the *para* position,
wherein each R⁶ stands independently for H or a linear or branched C₁ to C₂₀ alkyl group;
-N⁺R⁷R⁸R⁹ A⁻,
with
R⁷ = H, a linear or branched C₁ to C₂₀ alkyl group;
R⁸ = H, a linear or branched C₁ to C₂₀ alkyl group;
or R⁷ and R⁸ together with the nitrogen atom to which they are bonded, form a heteroaromatic ring system or an aromatic or non-aromatic ring;
R⁹ = absent, H, linear or branched C₁ to C₃₁ alkyl group;
A⁻ = an anion;
-P⁺R^{7'}R^{8'}R^{9'} A⁻,
with
R^{7'} = H, a linear or branched C₁ to C₂₀ alkyl group;
R⁸ = H, a linear or branched C₁ to C₂₀ alkyl group;
R^{9'} = absent, H, a linear or branched C₁ to C₃₁ alkyl group;
A⁻ = an anion; 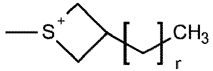 A⁻
with r = 5 to 30;
A⁻ = an anion; or
a quaternary alkyl phosphonium or alkyl sulfonium group, octenidine or a derivative thereof, chlorhexidine or another bisguanidine, triclosan or another chlorophenol, an inorganic group with antimicrobial properties, an antimicrobial peptide group;
Y = absent, O, S or COO;
Z = absent, O, S, NH, N⁺R³₂ or an amide group;
(b) at least one radically polymerisable monomer;
and
(c) at least one initiator for the radical polymerisation.

2. Dental material according to claim 1, wherein R¹ is a linear or branched C₅ to C₂₀ alkylene group and preferably a C₁₀ to C₂₀ alkylene group.

3. Dental material according to claim 1 or 2, wherein AG is a carboxylic acid group of the Formula

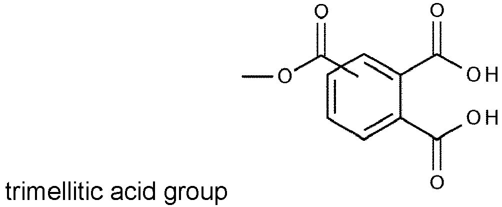

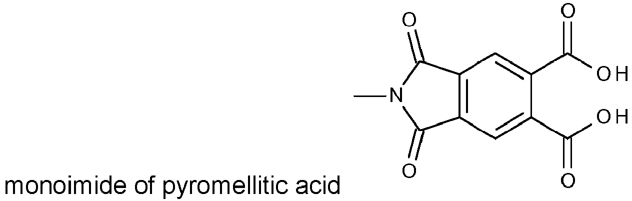

4. Dental material according to one of claims 1 to 3, wherein AG is a carboxylic acid anhydride group of the Formula

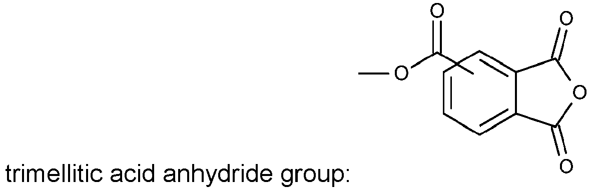

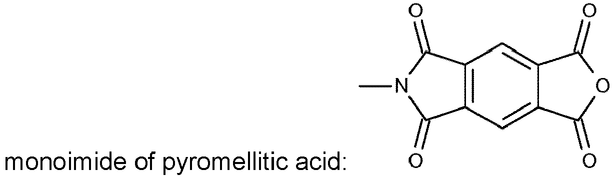

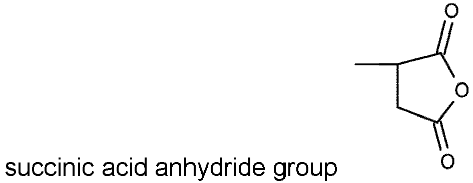

5. Dental material according to one of claims 1 to 4, wherein the antimicrobially active group WG is a quaternary alkylammonium group of the Formula 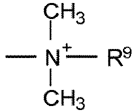 wherein R⁹ is a C₁ to C₃₁ alkyl, preferably C₅ to C₂₁ alkyl, in particular C₇ to C₁₈ alkyl, particularly preferably C₁₁ to C₁₆ alkyl and most preferably C₁₁ to C₁₄ alkyl, and most preferably an *N*,*N*-dimethyldodecylammonium group or *N,N-*dimethylcetylammonium group.

6. Dental material according to one of claims 1 to 5, wherein the spacer SP is a polyoxazoline group of the Formula: 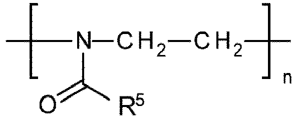 wherein n is preferably 3 to 500, preferably 5 to 200 and particularly preferably 10 to 100 and R⁵ is preferably -CH₃, -C₂H₅ or -C₃H₇.

7. Dental material according to one of claims 1 to 6, wherein at least one of the variables has the one of the following meanings:
m = 1;
p = 1:
R¹ = a C₁ to C₁₀ alkylene group and most preferably -CH₂-CH₂- or -CH₂-CH₂-NH-CH₂-CH₂-;
R² = phenylene or -CH₂-Ph-CH₂-;
R³ = methyl, ethyl, *n*-propyl or *i*-propyl, most preferably methyl;
SP = a polyoxazoline, polyester, polyamide, polyurea, polyurethane, polycyanoacrylate, polyacrylate or polymethacrylate group;
AG = a phosphonate, phosphate, carboxylic acid or carboxylic acid anhydride group;
WG = a pyridinium group of the Formulal 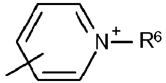 which is linked in the *ortho, meta* or *para* position and particularly preferably in the *para* position with the remainder of the compound of Formula (I), or
a pyridinium group of the Formula 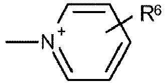 which may be substituted with R⁶ in the *ortho, meta* or *para* position and particularly preferably in the *para* position,
wherein each R⁶ stands independently for H or a linear or branched C₁ to C₂₀ alkyl group;
-N⁺R⁷R⁸R⁹ A⁻,
with
R⁷ = a C₁ to C₆ alkyl group, preferably -CH₃
R⁸ = a C₁ to C₆ alkyl group, preferably -CH₃
or R⁷ and R⁸ together with the nitrogen atom to which they are bonded, form a heteroaromatic ring system or an aromatic or non-aromatic ring of the Formula 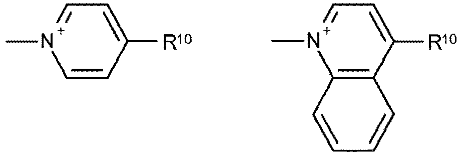 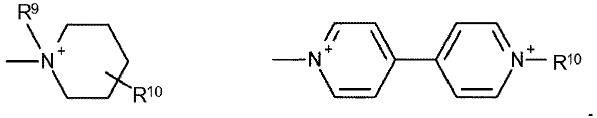
wherein R¹⁰ is a linear or branched C₁ to C₂₀ alkyl group;
R⁹ = -(CH₂)ᵣ-CH₃;
r = 5 to 30, preferably 10 to 25, particularly preferably 10 to 20;
A⁻ = F⁻, Cl⁻, Br⁻, I⁻, triflate (Trf), mesylate (Mes), tosylate (Tos), palmitate, stearate, gluconate, hexafluorophosphate, phenolate, (meth)acrylphosphonate, phosphate, hydrogen phosphate or dihydrogen phosphate, preferably F⁻, Cl⁻, Br⁻, I⁻, triflate (Trf), mesylate (Mes) or tosylate (Tos);
-P⁺R^{7'}R^{8'}R^{9'} A⁻,
with
R^{7'} = a C₁ to C₆ alkyl group, preferably -CH₃;
R^{8'} = -CH₃;
R^{9'} = -(CH₂)ᵣ-CH₃;
r = 5 to 30, preferably 10 to 25, particularly preferably 10 to 20;
A⁻ = F⁻, Cl⁻, Br⁻, I⁻, triflate (Trf), mesylate (Mes), tosylate (Tos), palmitate, stearate, gluconate, hexafluorophosphate, phenolate, (meth)acrylphosphonate, phosphate, hydrogen phosphate or dihydrogen phosphate, preferably F⁻, Cl⁻, Br⁻, I⁻, triflate (Trf), mesylate (Mes) or tosylate (Tos); 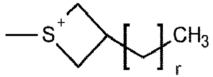 A⁻
with
r = 10 to 30, preferably 10 to 20;
A⁻ = F⁻, Cl⁻, Br⁻, I⁻, triflate (Trf), mesylate (Mes), tosylate (Tos), palmitate, tearate, gluconate, hexafluorophosphate, phenolate, (meth)acrylphosphonate, phosphate, hydrogen phosphate or dihydrogen phosphate, preferably F⁻, Cl⁻, Br⁻, I⁻, triflate (Trf), mesylate (Mes) or tosylate (Tos);
or
a quaternary alkyl phosphonium or alkyl sulfonium group, octenidine or a derivative thereof, chlorhexidine or another bisguanidine, triclosan or another chlorophenol, an inorganic group with antimicrobial properties which comprises one or more antimicrobially active metal ions, an antimicrobial magainin group or a lingual antimicrobial peptide (LPA) group;
Y = absent;
Z = absent, NH, or N⁺(CH₃)₂;
wherein all variables may be selected independently of each other.

8. Dental material according to one of claims 1 to 7, **characterised in that** it comprises
(a) 0.05 to 50 wt %, preferably 0.5 to 25 wt %, and particularly preferably 2 to 10 wt % of an antimicrobially active compound of the Formula (I);
(b) 5 to 95 wt %, preferably 5 to 85 wt %, and particularly preferably 5 to 70 wt % of a radically polymerisable monomer; and
(c) 0.01 to 5 wt % and preferably 0.1 to 5 wt % initiator for the radical polymerisation.

9. Dental material according to claim 8, **characterised in that** it additionally comprises (d) 5 to 60 wt %, preferably 5 to 50 wt %, and particularly preferably 5 to 45 wt % of a radically polymerisable acidic monomer; and/or
(e) 1 to 90 wt % and preferably 1 to 85 wt % filler and/or
(f) 0.5 to 99.95 wt %, preferably 0.5 to 60 wt % and particularly preferably 1 to 40 wt % solvent and/or
(g) 0.01 to 5 wt % and preferably 0.01 to 3.0 wt % of further additives.

10. Use of a dental material according to one of claims 1 to 9 for the production of an adhesive, primer, cement, coating material or filling material.

## Revendications

1. Matériau dentaire, qui contient
(a) au moins un composé à activité antimicrobienne, de formule (I)
[AG)ₘ-R¹-Z-SP-Y-R²-[WG]ₚ (I),
dans laquelle les variables ont les significations suivantes :
m = 1 ou 2 ;
p = 1 ou 2 ;
R¹ représente un radical alkylène en C₁-C₂₀ linéaire ou ramifié, qui peut être interrompu une ou plusieurs fois par O, S, NH, N⁺R³₂, SiR³₂, CONH, CONR³, COO et/ou OCONH ;
R² représente un radical aromatique en C₆-C₁₄ substitué ou non substitué ;
R³ représente chaque fois indépendamment un radical alkyle en C₁-C₂₀ linéaire ou ramifié ;
AG représente un groupe d'ancrage choisi parmi : 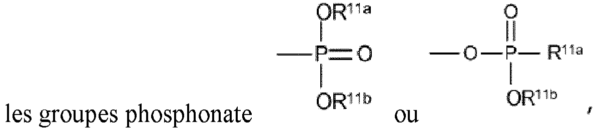 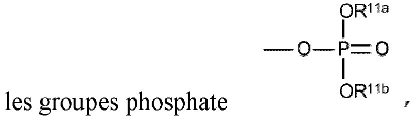 où R^{11a} et R^{11b} sont choisis indépendamment parmi un atome d'hydrogène, un groupe alkyle, aryle ou -Si(alkyle)₃, le groupe alkyle représentant de préférence un groupe méthyle, éthyle, n-propyle ou isopropyle, et où de façon particulièrement préférée R^{11a} et R^{11b} représentent chacun un groupe alkyle ou R^{11a} est un atome d'hydrogène et R^{11b} est un groupe alkyle, 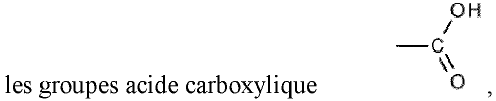 les groupes anhydride d'acide carboxylique 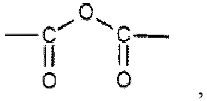
SP représente un espaceur polymère, qui est choisi parmi les groupes polyoxazoline, polyester, polyamide, polyurée, polyuréthane, polycyanoacrylate, polyacrylate et polyméthacrylate ;
WG représente un groupe à activité antimicrobienne, choisi parmi un groupe pyridinium de formule 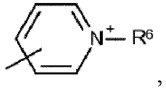 qui est lié en position ortho, méta ou para et de façon particulièrement préférée en position para au reste du composé de formule (I), ou
un groupe pyridinium de formule 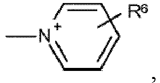 qui est substitué par R⁶ en position ortho, méta ou para et de façon particulièrement préférée en position para,
R⁶ représentant chaque fois indépendamment un atome d'hydrogène ou un radical alkyle en C₁-C₂₀ linéaire ou ramifié ;
un groupe -N⁺R⁷R⁸R⁹ A⁻, où
R⁷ représente un atome d'hydrogène, un radical alkyle en C₁-C₂₀ linéaire ou ramifié ;
R⁸ représente un atome d'hydrogène, un radical alkyle en C₁-C₂₀ linéaire ou ramifié ;
ou R⁷ et R⁸ forment ensemble, avec l'atome d'azote auquel ils sont liés, un système cyclique hétéroaromatique ou un cycle aromatique ou non aromatique ;
R⁹ est absent ou représente un atome d'hydrogène, un radical alkyle en C₁-C₃₁ linéaire ou ramifié ;
A⁻ représente un anion ;
un groupe -P⁺R^{7'}R^{8'}R^{9'} A⁻, où
R^{7'} représente un atome d'hydrogène, un radical alkyle en C₁-C₂₀ linéaire ou ramifié ;
R^{8'} représente un atome d'hydrogène, un radical alkyle en C₁-C₂₀ linéaire ou ramifié ;
R^{9'} est absent ou représente un atome d'hydrogène, un radical alkyle en C₁-C₃₁ linéaire ou ramifié ;
A⁻ représente un anion ;
un groupe 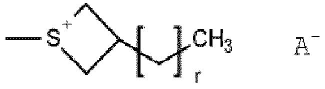 où
r = 5 à 30 ;
A⁻ représente un anion ; ou
un groupe alkylphosphonium ou alkylsulfonium quaternaire, l'octénidine ou un dérivé de celle-ci, la chlorhexidine ou une autre bisguanidine, le triclosan ou un autre chlorophénol, un groupe inorganique à propriétés antimicrobiennes, un groupe peptidique antimicrobien ;
Y est absent ou représente un atome d'oxygène ou de soufre ou un groupe COO ;
Z est absent ou représente un atome d'oxygène ou de soufre ou un groupe NH, N⁺R³₂ ou un groupe amido ;
(b) au moins un monomère polymérisable par voie radicalaire ; et
(c) au moins un amorceur pour la polymérisation radicalaire.

2. Matériau dentaire selon la revendication 1, dans lequel R¹ est un radical alkylène en C₅-C₂₀ linéaire ou ramifié et de préférence un radical alkylène en C₁₀-C₂₀.

3. Matériau dentaire selon la revendication 1 ou 2, dans lequel AG est un groupe acide carboxylique de formule 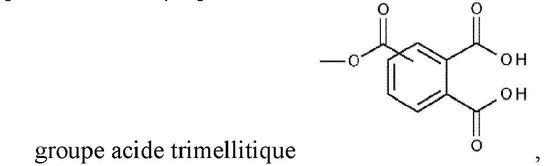 groupe monoimide d'acide pyromellitique

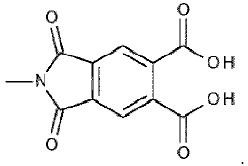

4. Matériau dentaire selon l'une quelconque des revendications 1 à 3, dans lequel AG est un groupe anhydride d'acide carboxylique de formule

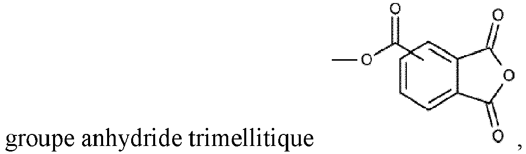

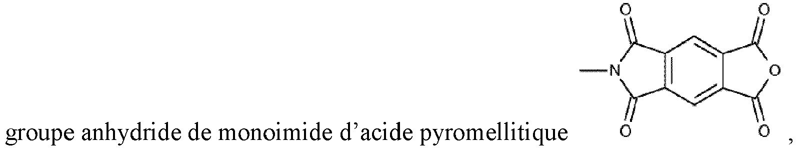

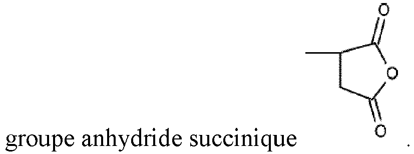

5. Matériau dentaire selon l'une quelconque des revendications 1 à 4, dans lequel le groupe à activité antimicrobienne WG est un groupe alkylammonium quaternaire de formule 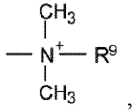 dans laquelle R⁹ est un groupe alkyle en C₁-C₃₁, de préférence un groupe alkyle en C₅-C₂₀, en particulier un groupe alkyle en C₇-C₁₈, de façon particulièrement préférée un groupe alkyle en C₁₁-C₁₆ et de façon tout particulièrement préférée un groupe alkyle en C₁₁-C₁₄, et de façon tout particulièrement préférée est un groupe N,N-diméthyldodécylammonium ou N,N-diméthylcétylammonium.

6. Matériau dentaire selon l'une quelconque des revendications 1 à 5, dans lequel l'espaceur SP est un groupe polyoxazoline de formule : 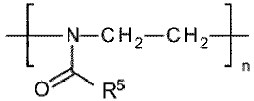 dans laquelle n vaut de préférence 3 à 500, de préférence 5 à 200 et de façon particulièrement préférée 10 à 100 et R⁵ représente de préférence un groupe -CH₃, -C₂H₅ ou -C₃H₇.

7. Matériau dentaire selon l'une quelconque des revendications 1 à 6, dans lequel au moins une des variables a l'une des significations suivantes :
m = 1;
p = 1;
R¹ représente un radical alkylène en C₁-C₁₀ et de façon tout particulièrement préférée -CH₂-CH₂- ou -CH₂-CH₂-NH-CH₂-CH₂- ;
R² représente un groupe phénylène ou -CH₂-Ph-CH₂- ;
R³ représente un groupe méthyle, éthyle, n-propyle ou isopropyle, de façon tout particulièrement préférée méthyle ;
SP représente un groupe polyoxazoline, polyester, polyamide, polyurée, polyuréthane, polycyanoacrylate, polyacrylate ou polyméthacrylate ;
AG représente un groupe phosphonate, phosphate, acide carboxylique ou anhydride d'acide carboxylique ;
WG représente un groupe pyridinium de formule 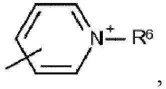 qui est lié en position ortho, méta ou para et de façon particulièrement préférée en position para au reste du composé de formule (I), ou
un groupe pyridinium de formule 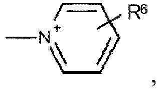 qui est substitué par R⁶ en position ortho, méta ou para et de façon particulièrement préférée en position para, R⁶ représentant chaque fois indépendamment un atome d'hydrogène ou un radical alkyle en C₁-C₂₀ linéaire ou ramifié ;
un groupe -N⁺R⁷R⁸R⁹ A⁻, où
R⁷ représente un radical alkyle en C₁-C₆, de préférence -CH₃ ;
R⁸ représente un radical alkyle en C₁-C₆, de préférence -CH₃ ;
ou R⁷ et R⁸ forment ensemble, avec l'atome d'azote auquel ils sont liés, un système cyclique hétéroaromatique ou un cycle aromatique ou non aromatique de formule 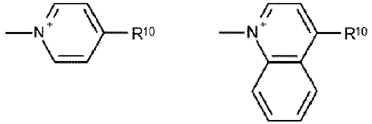 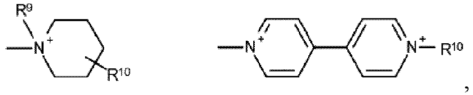
R⁹ R¹⁰ étant un radical alkyle en C₁-C₂₀ linéaire ou ramifié ; représente un groupe -(CH₂)ᵣ-CH₃ ;
r = 5 à 30, de préférence 10 à 25, de façon particulièrement préférée 10 à 20 ;
A⁻ représente un anion F⁻, Cl⁻, Br⁻, I⁻, triflate (Trf), mésylate (Mes), tosylate (Tos), palmitate, stéarate, gluconate, hexafluorophosphate, phénolate, (méth)acrylphosphonate, phosphate, hydrogénophosphate ou dihydrogénophosphate, de préférence F⁻, Cl⁻, Br⁻, I⁻-, triflate (Trf), mésylate (Mes) ou tosylate (Tos) ;
un groupe -P⁺R^{7'}R^{8'}R^{9'} A⁻, où
R^{7'} représente un radical alkyle en C₁-C₆, de préférence -CH₃ ;
R^{8'} représente un groupe -CH₃ ;
R^{9'} représente un groupe -(CH₂)ᵣ-CH₃ ;
r vaut de 5 à 30, de préférence 10 à 25, de façon particulièrement préférée 10 à 20 ;
A⁻ représente un anion F⁻, Cl⁻, Br⁻, I⁻ , triflate (Trf), mésylate (Mes), tosylate (Tos), palmitate, stéarate, gluconate, hexafluorophosphate, phénolate, (méth)acrylphosphonate, phosphate, hydrogénophosphate ou dihydrogénophosphate, de préférence F⁻, Cl⁻, Br⁻, I⁻, triflate (Trf), mésylate (Mes) ou tosylate (Tos) ;
un groupe 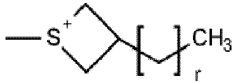 A⁻
où
r = 10 à 30, de préférence 10 à 20 ;
A⁻ représente un anion F⁻, Cl⁻, Br⁻, I⁻ , triflate (Trf), mésylate (Mes), tosylate (Tos), palmitate, stéarate, gluconate, hexafluorophosphate, phénolate, (méth)acryl-phosphonate, phosphate, hydrogénophosphate ou dihydrogénophosphate, de préférence F⁻, Cl⁻, Br⁻, I⁻, triflate (Trf), mésylate (Mes) ou tosylate (Tos) ; ou
un groupe alkylphosphonium ou alkylsulfonium quaternaire, l'octénidine ou un dérivé de celle-ci, la chlorhexidine ou une autre bisguanidine, le triclosan ou un autre chlorophénol, un groupe inorganique à propriétés antimicrobienne, qui contient un ou plusieurs ions(s) métallique(s) à activité antimicrobienne, un groupe magainine antimicrobien ou un groupe Lingual Antimicrobial Peptide (LAP) ;
Y est absent ;
Z est absent ou représente un groupe NH ou N⁺(CH₃)₂ ;
toutes les variables pouvant être choisies indépendamment les unes des autres.

8. Matériau dentaire selon l'une quelconque des revendications 1 à 7, qui contient
(a) 0,05 à 50 % en poids, de préférence 0,5 à 25 % en poids et de façon particulièrement préférée 2 à 10 % en poids de composé à activité antimicrobienne de formule (I) ;
(b) 5 à 95 % en poids, de préférence 5 à 85 % en poids et de façon particulièrement préférée 5 à 70 % en poids de monomère polymérisable par voie radicalaire ; et
(c) 0,01 à 5 % en poids et de préférence 0,1 à 5 % en poids d'amorceur pour la polymérisation radicalaire.

9. Matériau dentaire selon la revendication 8, qui contient en outre
(d) 5 à 60 % en poids, de préférence 5 à 50 % en poids et de façon particulièrement préférée 5 à 45 % en poids de monomère acide polymérisable par voie radicalaire et/ou
(e) 1 à 90 % en poids et de préférence 1 à 85 % en poids de charge et/ou
(f) 0,5 à 99,95 % en poids, de préférence 0,5 à 60 % en poids et de façon particulièrement préférée 1 à 40 % en poids de solvant et/ou
(g) 0,01 à 5 % en poids et de préférence 0,01 à 3,0 % en poids d'autres additifs.

10. Utilisation d'un matériau dentaire selon l'une quelconque des revendications 1 à 9 pour la fabrication d'un adhésif, primaire, ciment, matériau de revêtement ou matériau d'obturation.
